(19) Europäisches Patentamt
European Patent Office
Office européen des brevets

(11) EP 1 025 115 B1

(12) # FASCICULE DE BREVET EUROPEEN

(45) Date de publication et mention de la délivrance du brevet:
**04.06.2003 Bulletin 2003/23**

(21) Numéro de dépôt: **98946550.5**

(22) Date de dépôt: **02.10.1998**

(51) Int Cl.[7]: **C07H 19/04**, C07H 21/00, C12Q 1/68

(86) Numéro de dépôt international:
**PCT/FR98/02111**

(87) Numéro de publication internationale:
**WO 99/018114 (15.04.1999 Gazette 1999/15)**

(54) **NOUVEAUX CONJUGUES FLUORESCENTS DE NUCLEOSIDES OU DE NUCLEOTIDES, LEUR PROCEDE DE PREPARATION ET LEURS UTILISATIONS**

FLUORESZEND-KONJUGATEN VON NUKLEOSIDEN UND VON NUKLEOTIDEN, IHRER VERFAHREN ZUR HERSTELLUNG UND IHRER VERWENDUNGEN

NOVEL NUCLEOSIDE OR NUCLEOTIDE FLUORESCENT CONJUGATES, PREPARATION METHOD AND USES

(84) Etats contractants désignés:
**AT BE CH DE DK ES FI FR GB GR IE IT LI LU NL PT SE**

(30) Priorité: **03.10.1997 FR 9712379**

(43) Date de publication de la demande:
**09.08.2000 Bulletin 2000/32**

(73) Titulaire: **CIS BIO INTERNATIONAL**
**91400 Saclay (FR)**

(72) Inventeurs:
- **BAZIN, Hervé**
**F-30400 Villeneuve lès Avignon (FR)**
- **MATHIS, Gérard**
**F-30200 Bagnols-sur-Cèze (FR)**

(74) Mandataire: **Gillard, Marie-Louise et al**
**Cabinet Beau de Loménie**
**158, rue de l'Université**
**75340 Paris Cédex 07 (FR)**

(56) Documents cités:
**EP-A- 0 180 492**
**EP-A- 0 321 353**
**WO-A-92/01225**
**WO-A-92/13264**
**WO-A-92/14841**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention concerne de nouveaux conjugués fluorescents de nucléosides ou de nucléotides, utilisables notamment pour détecter, localiser et/ou isoler des acides nucléiques ou des molécules d'intérêt biologique ou clinique à structure nucléosidique ou susceptibles d'intéragir avec des acides nucléiques.

Dans la suite de la description, les abréviations suivantes seront utilisées :

ARN : acide ribonucléique
ADN : acide désoxyribonucléique
A : Adénosine
C : Cytidine
G : Guanosine
T : Thymidine
U : Uridine
I : Inosine
suffixe MP : monophosphate
suffixe DP : diphosphate
suffixe TP : triphosphate
préfixe d : désoxy.

Une réaction de synthèse enzymatique d'ADN emploie une matrice d'ARN ou d'ADN, une amorce oligonucléotidique de séquence complémentaire à un segment de la matrice, une enzyme appropriée et quatre désoxynucléotides dATP, dCTP, dGTP et dTTP (ou dUTP). Diverses enzymes sont connues, comme E. Coli DNA polymérase, T7 DNA polymérase, le fragment de Klenow de la DNA polymérase, la Taq DNA polymérase, une transcriptase inverse, auxquelles on peut ajouter la terminal nucléotidyl transférase qui présente la particularité de ne pas nécessiter de matrice. La synthèse d'ARN se fait de façon similaire sinon que les amorces et matrices requises sont différentes et que des ribonucléotides (ATP, CTP, GTP et UTP) sont utilisés en présence de RNA polymérases.

Les nucléotides ou polynucléotides peuvent être marqués radioactivement ($^{3}H$, $^{32}P$, $^{14}C$, $^{35}S$ ou $^{125}I$).

L'utilisation de molécules marquées présente les inconvénients classiquement associés avec les isotopes radioactifs qui sont les risques liés à la radioactivité ainsi qu'un stockage et une disponibilité limités due à la décroissance radioactive et aux phénomènes de radiolyse.

Le marquage chimique au niveau des nucléotides ou des polynucléotides, permettant d'éviter ces inconvénients, a été décrit dans la littérature. Notamment, on a décrit le marquage par de la biotine de nucléotides dérivés du dUTP ou de l'UTP (Langer P.R., Waldrop A.A., Ward D.C., 1981; Proc. Natl. Acad. Sci. USA, 78, 6633-37). Ce sont des dérivés dans lesquels la biotine est liée à la position C-5 du noyau pyrimidine par un bras alkylamine. Ces nucléotides marqués sont incorporés in vitro dans des polynucléotides par l'action de polymérases à ADN ou à ARN (EP 0 063 879) et permettent une détection colorimétrique d'acides nucléiques par une réaction de «dot-blot» (Leary J.J., Brigati D.J. and Ward D.D.; 1983; Proc. Natl. Acad. Sci. USA, 80, 4045-49).

D'autres analogues de nucléotides marqués à la biotine basés sur des dérivés de la N-4-aminoalkyl-désoxycytidine et de la N-6-aminoalkyl-désoxyadénosine sont décrits dans le brevet US 4,828,979 et dans Gebeyehu G.G. *et al.* Nucleic. Acids. Res; 1987, 15, 4513-4534.

Des dérivés de dUTP, de dATP substitué par de la biotine en position C-8, ainsi que la possibilité de substitution en C-7 d'une 7-déazapurine sont également décrits (EP 0 063 879).

Le dérivé Bio-15-dGTP a également été décrit (Gilliam I.C. and Tener G.M. ; 1989 ; Nucleoside & Nucleotide ; 8, 1453-62). Des dérivés fluorescents tels que la fluorescéine ou la rhodamine peuvent être incorporés dans un acide nucléique par l'intermédiaire d'un nucléoside triphosphate (dATP, dUTP, dCTP) marqué (WO 93 19206). Une discussion sur la position des substitutions sur les purines et pyrimidines ainsi que sur la nature des bras espaceurs utilisables pour le marquage des didésoxynucléotides avec des dérivés de la fluorescéine, bien que dédiée aux terminateurs de chaîne pour le séquençage, donne une vue d'ensemble de la chimie des nucléotides marqués (Confalone P.T., 1990, J. Heterocydic Chem., 27, 31-46).

L'utilisation conjointe de nucléosides triphosphate marqués à l'aide de traceurs différents (Biotine-11-dUTP, dig-11-dUTP et FITC-11dUTP) permet une visualisation simultanée de séquences différentes lors d'une hybridation (RIED T. et al. Proc. Natl. Acad. Sci. USA (1992), 89, 1388-1392).

L'incorporation de désoxynucléotides marqués à la fluorescéine tels que Fl-dUTP ou Fl-dCTP est également effectuée en ne substituant que partiellement le nucléotide naturel par le composé marqué, dCTP / FldCTP ≈ 2:1 (WO93/19206). Ce même brevet décrit que par un choix de l'enzyme et des conditions expérimentales, il est possible de substituer la totalité d'un nucléotide par un nucléotide marqué.

Les données de la littérature montrent que l'efficacité d'incorporation d'un conjugué de triphosphate est modifiée par le couplage d'une molécule comme la biotine et dans une moindre mesure par la présence du bras permettant le couplage.

Par exemple, dans une réaction de «nick-translation» en présence d'une polymérase à ADN, les taux d'incorporation de divers analogues de triphosphates ont été comparés au nucléoside naturel pris comme référence (100 % d'incorporation) pour un même temps de réaction (90 min) (Gebeyehu G. *et al*. Nucleic Acids Res., 1987, **15**, 4525).

Les molécules conjuguées à des triphosphates de nucléosides (Goodchild, J. Bioconjugate chem., 1990, p171 ; Kricka J. Non isotopic Blotting, and Sequencing 1995 Academic Press p47 ; Zhu Z. et al. Nucleic Acids Res., 1994, 3418-3422) sont des molécules de taille relativement petites (<800Da), soit neutres soit chargées négativement et de forme essentiellement plane et par conséquent, leur encombrement est réduit mais suffisant pour perturber l'incorporation enzymatique du nucléoside triphosphate.

Des cryptates de terre rare utilisables comme marqueurs pour la détection de substances biologiques sont décrits dans la demande EP 0 321 353.

La demande WO92/14841 décrit des conjugués constitués d'un chélate de terre rare comprenant n unités triazine liées de manière covalente à un oligonucléotide comprenant au moins 4 + n désoxy- ou ribo-nucléotides.

On a maintenant trouvé de nouveaux conjugués de nucléosides ou de nucléotides comprenant :

- un ribo- ou désoxyribo-nucléoside ou nucléotide natif ou chimiquement modifié ou conjugué à une ou plusieurs molécules marqueur(s), dont au moins un atome de carbone du cycle ou un atome d'azote exocyclique du cycle purique ou pyrimidique ou encore un atome de carbone du cycle pentofuranose est susceptible d'être impliqué dans une liaison avec un marqueur fluorescent, et
- au moins un marqueur fluorescent lié au(x)dit(s) atome(s) constitué par un cryptate de terre rare.

Dans un aspect préféré, lesdits conjugués comprennent :

- un ribo- ou désoxyribo-nucléoside ou nucléotide natif ou chimiquement modifié ou conjugué à une ou plusieurs molécules marqueur(s), dont au moins un atome de carbone du cycle ou d'azote exocyclique du cycle purique ou pyrimidique est susceptible d'être impliqué dans une liaison avec un marqueur fluorescent, et
- au moins un marqueur fluorescent lié au(x)dit(s) atome(s) constitué par un cryptate de terre rare.

Lesdits conjugués peuvent être utilisés dans toutes les applications des nucléosides ou nucléotides marqués sans présenter d'inconvénients majeurs d'utilisation et présentent notamment une capacité élevée à être incorporés dans de l'ADN simple brin ou double brin.

Ces propriétés sont d'autant plus surprenantes que les cryptates de terre rare sont des molécules de masse moléculaire élevée (supérieure à 1400 Da), possédant une structure tridimensionnelle qui présente plus d'encombrement stérique qu'une molécule globalement plane, et ayant un caractère ionique provenant de la présence de l'ion complexé qui leur confère une charge positive.

Du fait de ces caractéristiques structurales, on pouvait s'attendre à ce que les charges positives d'un cryptate de terre rare interagissent fortement avec les charges négatives du groupe triphosphate et modifient sa réactivité ainsi que les qualités de fluorescence du cryptate.

Egalement, l'activité des enzymes telles que les polymérases étant sensibles à la présence et à la concentration de certains ions ou agents complexants dans le milieu réactionnel, on pouvait s'attendre à ce que les conjugués selon l'invention entraînent une forte diminution de l'incorporation des nucléotides triphosphates, et donc un faible rendement, notamment lors d'une synthèse de polynucléotides.

De manière surprenante, les résultats obtenus en utilisant les conjugués selon l'invention montrent que, non seulement les cryptates de terre rare n'ont pas d'influence défavorable dans les applications qui font intervenir des réactions enzymatiques, mais conservent leurs propriétés fluorescentes intrinsèques.

De manière avantageuse, on a également trouvé que les conjugués selon l'invention présentaient de nouvelles caractéristiques de fluorescence et, en particulier, que la durée de vie d'émission de l'ion de terre rare complexé était significativement augmentée.

Lesdits conjugués peuvent donc être utilisés à titre de marqueurs fluorescents dans toutes les utilisations dans lesquelles on effectue une détection quantitative ou qualitative par mesure de la fluorescence directe ou indirecte.

Dans un aspect préféré, l'invention concerne des conjugués fluorescents de nucléotides comprenant un ribonucléotide choisi parmi AMP, ADP, ATP, GMP, GDP, GTP, CMP, CDP, CTP, UMP, UDP, UTP, TMP, TDP, TTP, 2Me AMP, 2Me ADP, 2Me ATP, 1Me GMP, 1Me GDP, 1Me GTP, 5Me CMP, 5Me CDP, 5Me CTP, 5MeO CMP 5MeO CDP, 5MeO CTP, 7-déaza-ATP, 7-déaza-GTP ou, le cas échéant, un désoxyribo-nucléotide choisi parmi les désoxy- ou didésoxyribonucléotides correspondants à ces ribo-nucléotides, et en particulier

- les dérivés de la 2'-désoxy-uridine-5'-triphosphate ou de l'uridine-5'-triphosphate fonctionnalisés en position 5 de

la base (principalement squelette aminoallyle ou aminopropyne),

- les dérivés de la 2'-désoxy-cytidine-5'-triphosphate ou de la cytidine-5'-triphosphate fonctionnalisés en position 4 ou 5 de la base (principalement squelette aminoallyle ou aminopropyne pour la position 5),
- les dérivés de la 2'-désoxy-adénosine-5'-triphosphate ou de l'adénosine-5'-triphosphate fonctionnalisés en position 6 ou 8 de la base,
- les dérivés de la 2'-désoxy-guanosine-5'-triphosphate ou de la guanosine-5'-triphosphate fonctionnalisés en position 6 ou 8 de la base,
- les dérivés de la 2'-désoxy-7-déaza-adénosine-5'-triphosphate ou de la 7-déaza-adénosine-5'-triphosphate fonctionnalisés en position 7 de la base, et
- les dérivés de la 2'-désoxy-7-déaza-guanosine-5'-triphosphate ou de la 7-déaza-guanosine-5'-triphosphate fonctionnalisés en position 7 de la base.

Les nucléotides utilisables aux fins de l'invention comprennent également des nucléotides modifiées chimiquement sur la partie triphosphate, en particulier les dérivés $\alpha$-thiotriphosphates.

Selon un autre aspect, l'invention concerne des conjugués fluorescents de nucléosides dans lesquels le ribo- ou désoxyribo-nucléoside est choisi parmi A, G, C, U, T, les désoxy- ou didésoxynucléosides correspondants, et leurs analogues chimiquement modifiés, et en particulier la 3'-azido-3'-désoxythymidine et ses dérivés ; et les analogues 2', 3'-didéoxy de A, T, C, G, U, I.

Le marqueur fluorescent est constitué par un cryptate de terre rare choisi de préférence parmi les cryptates de terbium, d'europium, de samarium ou de dysprosium.

Dans la suite de la description, la notion de «cryptate» ainsi que la nomenclature des macrocycles et polycycles utilisables sont telles que définies par J.M. Lehn dans Struct. Bonding (Berlin), 16, 1, 1973 et dans Acc. Chem. Res. 11, 49 (1978).

Selon un aspect préféré, ledit cryptate de terre rare est constitué d'au moins un sel de terre rare complexé par un composé macropolycyclique de formule

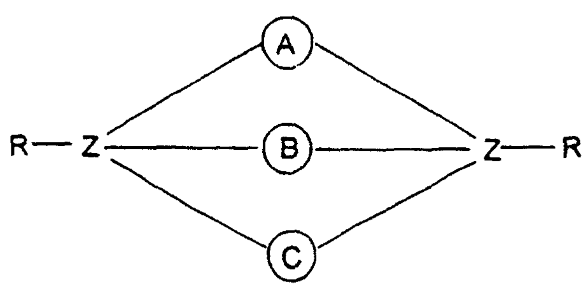

I

dans laquelle Z est un atome ayant 3 ou 4 valences, R est rien ou représente l'hydrogène, le groupe hydroxy, un groupe amino ou un radical hydrocarboné, les radicaux bivalents Ⓐ , Ⓑ et ©, sont indépendamment l'un de l'autre des chaînes hydrocarbonées qui contiennent éventuellement un ou plusieurs hétéroatomes et sont éventuellement interrompues par un hétéromacrocycle, au moins l'un des radicaux Ⓐ , Ⓑ et © comportant de plus au moins un motif moléculaire ou étant essentiellement constitué par un motif moléculaire, ledit motif moléculaire possédant une énergie de triplet supérieure à celle du niveau émissif de l'ion de terre rare complexé.

De préférence, il s'agit d'un cryptate constitué d'au moins un sel de terre rare complexé par un composé macropolycyclique de formule (I) ci-dessus, dans laquelle le motif moléculaire est choisi parmi la phénanthroline, l'anthracène, le benzène, le naphtalène, les bi- et ter-phényle, l'azobenzène, l'azopyridine, la pyridine, les bipyridines, les bisquinoléines et les composés de formules ci-après :

$$- C_2H_4 - X_1 - C_6H_4 - X_2 - C_2H_4 -$$

$$- C_2H_4 - X_1 - CH_2 - C_6H_4 - CH_2 - X_2 - C_2H_4 -$$

$X_1$ et $X_2$ pouvant être identiques ou différents désignent l'oxygène, l'azote ou le soufre,

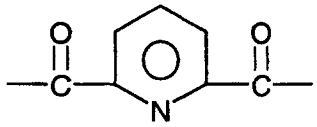

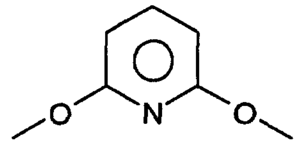

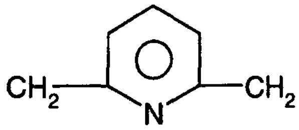

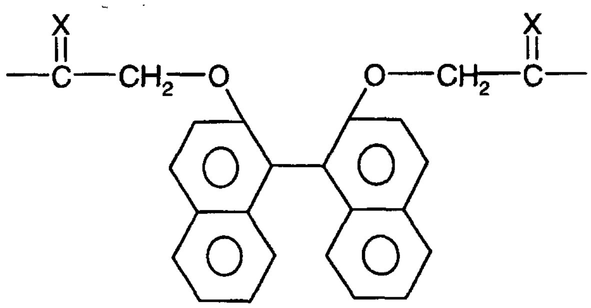

X étant l'oxygène ou l'hydrogène.

Dans un aspect avantageux, le marqueur fluorescent est un cryptate de terre rare constitué de l'ion terbium ou europium complexé par l'un des composés macrocycliques ci-après :

(22)phénanthroline ; (22)phénanthroline amide ; (22)anthracène ; (22)anthracène amide ; (22)bi-isoquinoléine; (22)biphényl-bis-pyridine ; (22)bipyridine; (22)bi-pyridine amide; les macropolycycles tris-bipyridine, tris-phénanthroline, phénanthroline-bis-bipyridine, bi-isoquinoléine-bis-bipyridine, bis-bipyridine diphénylbipyridine.

Un marqueur particulièrement avantageux est le cryptate d'europium Eu tris bipyridine.

De tels composés sont par exemple décrits dans le brevet EP 180 492.

On peut également utiliser des composés macropolycycliques cryptates complexant des ions de terre rare dans lesquels le motif moléculaire est choisi parmi les bipyrazines, les bipyrimidines et les hétérocycles azotés comportant des groupes N-oxydes.

Des composés macropolycycliques à unités bipyrazines sont décrits dans F. Bodar-Houillon et al., New J. Chem., 1996, 20, 1041-1045.

Des composés macropolycycliques à unités bipyrimidines sont décrits dans J. M. Lehn et al., Helv. Chim. Acta, 1992, 75, 1221.

Des composés macropolycycliques comprenant des hétérocycles azotés comportant des groupes N-oxydes sont décrits dans J.M. Lehn et al., Helv. Chim. Acta, 1991, 74, 572.

Le cryptate de terre rare utilisé comme marqueur fluorescent peut également être constitué d'au moins un sel de terre rare complexé par un composé macropolycyclique répondant à l'une des formules II ou III ci-après :

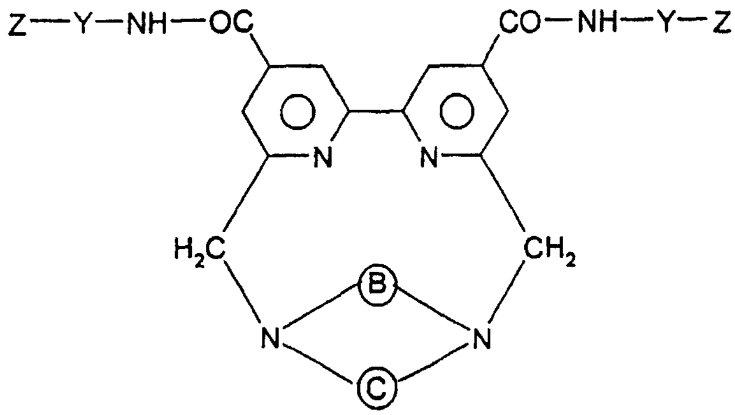

II

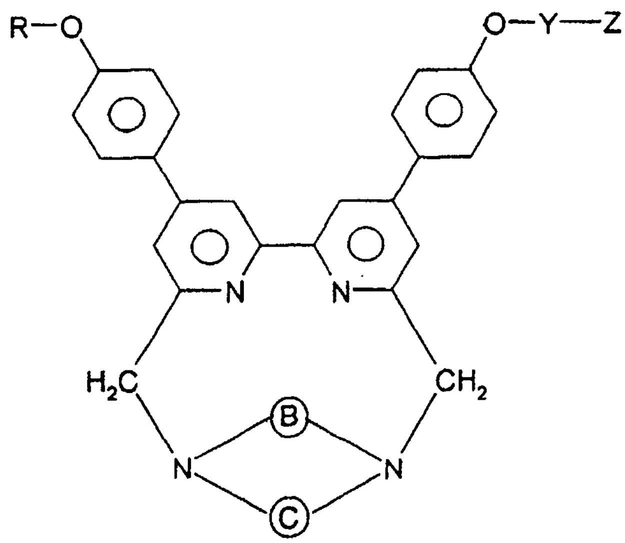

III

dans lesquels :

- le cycle de formule

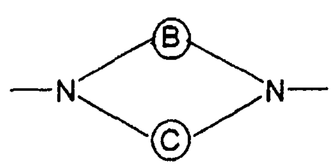

  est l'un des cycles suivants :

1) 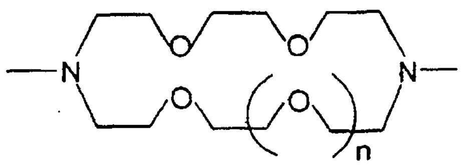

n = 0 ou 1
macrocycle [$N_2O_4$] ou cycle (22)
macrocycle [$N_2O_3$] ou cycle (21)

2) 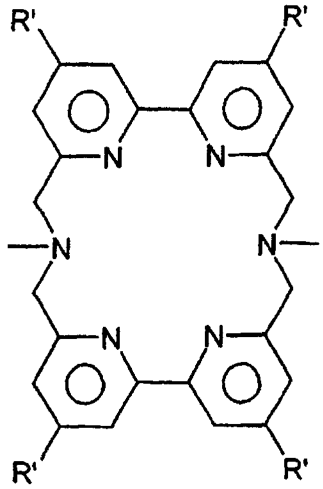

macrocycle bis-bipyridine

- Y est un groupe ou un bras d'espacement qui est constitué par un radical organique bivalent, choisi parmi les groupes alkylène linéaires ou ramifiés en $C_1$ à $C_{20}$ contenant éventuellement une ou plusieurs doubles liaisons et/ou contenant éventuellement un ou plusieurs hétéroatomes tels que l'oxygène, l'azote, le soufre, le phosphore ou un ou plusieurs groupe(s) carbamoyle ou carboxamido ; parmi les groupes cycloalkylène en $C_5$ à $C_8$ ou parmi les groupes arylène en $C_8$ à $C_{14}$, lesdits groupes alkylène, cycloalkylène ou arylène étant éventuellement substitués par des groupes alkyle, aryle ou sulfonate ;
- Z est un groupe fonctionnel susceptible de se lier de façon covalente avec une substance biologique ;
- R est un groupe méthyle ou représente le groupe -Y-Z ;
- R' est l'hydrogène ou un groupe -COOR" dans lequel R" est un groupe alkyle en $C_1$ à $C_{10}$ et représente de préférence le groupe méthyle, éthyle ou tertiobutyle ou bien R' est un groupe -CO-NH-Y-Z.

De tels composés sont décrits par exemple dans le brevet EP 321 353.

Au sein des conjugués selon l'invention, ledit marqueur fluorescent peut être lié au ribo- ou désoxyribo-nucléoside ou nucléotide soit directement, soit par l'intermédiaire d'un bras d'espacement.

Par « liaison directe », on entend la liaison du marqueur fluorescent sur un groupe fonctionnel préalablement introduit ou généré sur un ou plusieurs atomes de la base ou de l'unité pentofuranose du ribo- ou désoxyribo-nucléoside ou nucléotide.

Dans la présente description, on désigne par groupe fonctionnel toute fonction portée par la partie nucléosidique ou nucléotidique ou introduite sur cette partie par toute méthode connue par l'homme du métier et capable de se lier par liaison covalente, directement ou après activation avec une fonction présente sur le cryptate ou sur le bras espaceur porté par le cryptate. De tels groupes fonctionnels sont notamment les fonctions $NH_2$, COOH, CHO, OH ou SH ainsi que les fonctions capables de donner des liaisons covalentes par substitution (halogénures, sulfonates, époxyde) ou par addition (double liaisons type maléïmide). Ces fonctions sont généralement portées par une chaîne hydrocarbonée elle-même reliée à la partie nucléosidique ou nucléotidique.

Des méthodes d'introduction de ces groupes fonctionnels sont notamment décrites dans C. Kessler, Nonisotopic probing, Blotting and Sequencing, 2nd edition, L.J. Kricka (1995), Ed. Academic press Ltd., Londres, p. 66-72.

Ce bras d'espacement est par exemple constitué par un radical organique bivalent, choisi parmi les groupes alkylène linéaires ou ramifiés en $C_1$-$C_{20}$, contenant éventuellement une ou plusieurs doubles liaisons ou triples liaisons et/ou contenant éventuellement un ou plusieurs hétéroatomes, tels que l'oxygène, l'azote, le soufre, le phosphore ou

un ou plusieurs groupe(s) carbamoyle ou carboxamido ; les groupes cycloalkylène en $C_5$-$C_8$ et les groupes arylène en $C_6$-$C_{14}$, lesdits groupes alkylène, cycloalkylène ou arylène étant éventuellement substitués par des groupes alkyle, aryle ou sulfonate.

En particulier, il peut être choisi parmi les groupes :

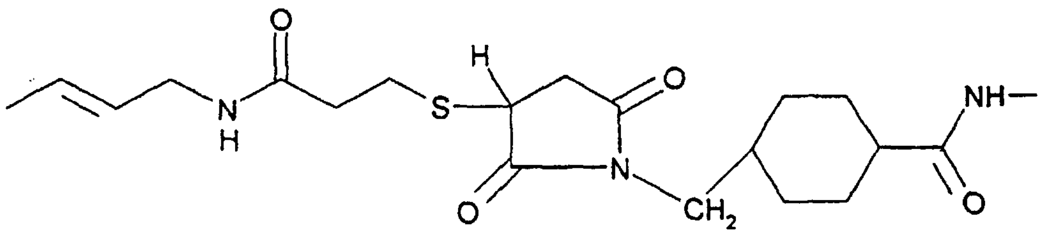

et

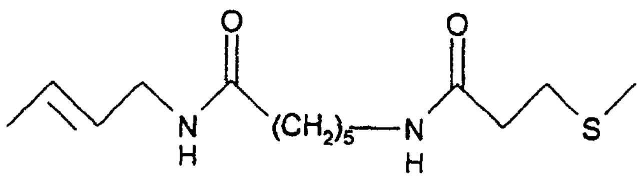

Dans un aspect préféré, le conjugué selon l'invention comprend un désoxyribonucléotide qui est la désoxyuridine, un marqueur fluorescent qui est le cryptate d'europium Eu trisbipyridine et un bras d'espacement qui est un groupe 3-aminoallyle.

L'invention concerne également, selon un aspect ulltérieur, un procédé de préparation des conjugués décrits plus hauts.

Ledit procédé de préparation est caractérisé en ce qu'on fait réagir un ribo- ou désoxyribo-nucléoside ou nucléotide natif ou chimiquement modifié ou conjugué à une ou plusieurs molécules marqueur(s), dont au moins un atome de carbone du cycle ou un atome d'azote exocyclique du cycle purique ou pyrimidique, ou encore un atome de carbone de l'unité pentofuranose est susceptible d'être impliqué dans une liaison avec un marqueur fluorescent avec au moins un marqueur fluorescent lié au(x)dit(s) atome(s) constitué par un cryptate de terre rare, et qu'on isole le conjugué ainsi obtenu.

Les ribo- ou désoxyribo-nucléosides ou nucléotides ainsi que les marqueurs fluorescents utilisables dans ce procédé de préparation sont tels que décrits plus haut.

Les conjugués selon l'invention qui comprennent un ribo- ou un désoxyribonucléotide sont particulièrement adaptés pour toutes les applications qui nécessitent une mesure qualitative ou quantitative lors de la synthèse ou de l'utilisation de polynucléotides.

Selon un de ses aspects, l'invention concerne également les polynucléotides comprenant au moins un conjugué fluorescent de ribo- ou de désoxyribo-nucléotide tel que décrit plus haut en tant que nucléotide constitutif.

Avantageusement, les polynucléotides obtenus par incorporation de conjugués selon l'invention peuvent comprendre un nombre de conjugués supérieur à 1, et donc un nombre de marqueurs fluorescents supérieur à 1.

En utilisant les conjugués selon l'invention, il est ainsi possible de réaliser le polymarquage de polynucléotides par voie enzymatique. Cette technique permet d'obtenir des polynucléotides marqués plus facilement et en assurant une meilleure reproductibilité qu'en utilisant les techniques classiques de marquage fluorescent par voie chimique. En particulier, elle évite les étapes de séparation nécessaires pour éliminer du milieu les polynucléotides et/ou les marqueurs fluorescents fonctionnalisés n'ayant pas réagi.

On notera que si l'on utilise des cryptates bi-fonctionnalisés, la conjugaison ne se fait que sur un bras, ce qui permet toujours l'incorporation du conjugué dans le cadre d'une synthèse de polynucléotides.

Les conjugués selon l'invention qui comprennent un ribo- ou un désoxyribonucléotide peuvent être utilisés pour la détection et/ou la localisation de composés contenant au moins une séquence d'acide nucléique.

Parmi ces utilisations, on peut citer de manière non limitative :

- la détection et/ou la localisation de séquences spécifiques d'ADN, par exemple en vue de l'établissement de la

cartographie de chromosomes ou la détection d'une mutation ;
- la synthèse de sondes utilisables en recherche biomédicale ou pour l'établissement d'un diagnostic clinique.

On peut également les utiliser dans un procédé de mesure de l'activité enzymatique d'une enzyme impliquée dans une réaction de synthèse d'acide nucléique, par exemple une activité polymérase à ADN ou à ARN, transcriptase inverse, transférase ou ligase, dans laquelle on mesure la fluorescence émise directement ou indirectement par ledit conjugué, ladite émission de fluorescence étant corrélée au taux d'incorporation dudit conjugué dans le polynucléotide d'acide nucléique synthétisé.

Les conjugués selon l'invention peuvent également être utilisés pour mesurer l'activité enzymatique d'une enzyme ayant pour substrat un acide nucléique, par exemple une activité phosphodiestérase, DNAse ou Rnase, la fluorescence émise directement ou indirectement par ledit conjugué étant corrélée soit à ladite activité, soit à l'inhibition de ladite activité.

On peut également les utiliser pour mesurer une activité enzymatique modifiant la structure d'un acide nucléique telle qu'une activité hélicase ou intégrase ou une activité modifiant la topologie d'un acide nucléique telle qu'une activité topoisomérase.

Les conjugués selon l'invention peuvent également être utilisés à titre de marqueurs, par exemple pour la préparation d'un composé comprenant un acide nucléique dans lequel est incorporé ledit conjugué en vue d'une détection.

La fluorescence émise par les conjugués selon l'invention peut être soit «directe» : le signal lumineux est émis par le conjugué après excitation à une longueur d'onde donnée, soit « indirecte » : l'émission du signal lumineux est induite par un transfert d'énergie non radiatif entre le conjugué après excitation dit « composé donneur » et une autre molécule fluorescente dite « composé accepteur ».

Dans ce cas particulier, les conditions suivantes sont remplies :

- d'une part, le composé fluorescent accepteur possède un spectre d'absorption qui recouvre au moins partiellement le spectre d'émission du donneur et présente une absorbance molaire élevée dans cette zone de recouvrement, et un spectre d'émission dans une gamme de longueur d'ondes où le donneur présente une émission intrinsèque faible ;
- d'autre part, l'accepteur et le donneur se situent à proximité l'un de l'autre, l'orientation de leurs dipoles de transition étant approximativement parallèles.

Le principe de la technique de transfert d'énergie non radiatif est décrit notamment dans G. Mathis et al., Clin. Chem., 1993, 39, 1953-1959.

L'invention est illustrée par les exemples ci-après, dans lesquels certaines concentrations sont données en unité d'absorption (UA) à une longueur d'onde donnée -(exprimée en nm) par unité de volume (exprimée en ml) et exprimées par le même chiffre que la densité optique de la solution concernée.

EXEMPLE 1: Synthèse de la désoxyuridine marqué au cryptate d'europium trisbipyridine (conjugué K-11-dUTP).

Dans ce conjugué, le chiffre 11 indique le nombre total d'atomes du bras espaceur et du groupement fonctionnel qui relie la structure cryptate au nucléotide (ici, la liaison se fait en position 5 de la pyrimidine).

Le nucléoside-triphosphate utilisé est le 5-[N-(6-aminocaproyl)-3-aminoallyl]-2'-désoxyuridine-5'-triphosphate] (AH-dUTP) préparé par réaction du trifluoroacétamido-caproate de N-hydroxysuccinimide (M.S Urdea et al., Nucleic acids Res., 1988, 4937) sur le 5-(3-aminoallyl)-2'-désoxyuridine-5'-triphosphate préparé suivant un procédé de la littérature (Langer et al. Proc. Natl. Acad. Sci. USA (1981), 78, 6633-6637), suivi d'une déprotection ammoniacale ($NH_4OH$ aqueuse 3 %, 45 min à 60°C). Le composé est purifié sur DEAE-Sepharose ® (Pharmacia) dans un gradient linéaire d'hydrogénocarbonate de triéthylammonium (0,1 M à 0,3 M).

1) Méthode A

On dilue 68 µl d'une solution d'AH-dUTP à 6 µmole/ml (soit 0,4 µmole) à 250 µl d'hydrogénocarbonate de triéthylammonium (TEAB) 0,1 M pH 7,8 et on ajoute 320 µl d'une solution de cryptate de N-hydroxysuccinimide (4 mg/ml dans l'acétonitrile) préparée comme suit. Le cryptate d'Europium [(bis-bpy)-(bpy-diester)] décrit dans l'exemple 4, section A, de la demande EP 0 321 353 est hydrolysé par NaOH et le cryptate diacide obtenu est purifié sur colonne RP-18 HPLC (gradient d'acétonitrile dans de l'acide trifluoroacétique à 1% dans l'eau). Le cryptate d'Europium [(bis-bpy)-(bpy-diacide)] ainsi obtenu (4mg) est dissout dans 0,5 ml d'acétonitrile anhydre et on ajoute 1mg de N-hydroxysuccinimide, puis une solution de 1,9 mg de dicyclohexylcarbodiimide dissout dans 0,5 ml d'acétonitrile. Après 16 h de réaction, le précipité de dicyclohexylurée est filtré et la solution de cryptate de N-hydroxysuccinimide est utilisée

directement pour le couplage

Après 45 min sous agitation, on ajoute 15 µl de TEAB 1M pH 8,5 puis on injecte le mélange sur une colonne Superdex 30 ® HR 10/30 (Pharmacia) en éluant par du TEAB 0,05M pH 7 contenant 10 % d'acétonitrile (débit 1 ml/min).

On collecte le composé de Rt ≅16,4 mn, puis cette fraction dénommée fraction 1 est concentrée sous vide (speed-vac) jusqu'à un volume de 350 µl et contient 8 $UA_{304}$ nm. En estimant un $\varepsilon_{304} \cong 35000$, on estime que la concentration en K-dUTP est d'environ 0,72 mM.

Une aliquote (90 µl) de cette fraction 1 est injectée sur la même colonne éluée par un tampon triéthylammonium acétate 25 mM pH 7 contenant 5 % d'acétonitrile. La fraction correspondant au seul pic du chromatogramme est collectée (16 min < Rt < 19 min) et concentrée sous vide (speed-vac). On obtient 150 µl d'une solution de K-dUTP dénommée fraction 2 contenant 1,95 $UA_{304}$ nm.

Le composé est analysé en spectrométrie de masse (électrospray mode positif) :

$$(M\text{-}2H)^+ = 1431$$

$$(M\text{-}2H+CH_3COOH)^+ = 1491.$$

Le spectre UV dans l'eau présente un maximum à 241 nm caractéristique de la partie nucléosidique de la molécule (λmax = 289 nm, ε = 7100, λmax = 240 nm ε = 10700) et un maximum à 304 nm proche du λmax de 305 nm (ε = 30000) caractéristique du cryptate d'Europium. On observe un rapport $A_{304}/A_{241} \cong 0,83$ compatible avec la structure proposée.

2) <u>Méthode B</u>

On dissout 0,08 µmol d'une solution d'AH-dUTP dans 80 µl de tampon borate 0,1 M pH 8,6 et on ajoute 90 µl d'une solution de cryptate de N-hydroxysuccinimide (4 mg/ml) préparée comme décrit ci-dessus dans la méthode A.

Après 60 min à 20°C, on ajoute 5 µl de TEAB 1M pH 8,5 et 45 µl d'$H_2O$. On injecte la totalité du mélange réactionnel sur une colonne Superdex peptide HR 10/30 (Pharmacia) en éluant par du TEAB 0,05M pH 7 contenant 10 % d'acétonitrile (débit 1 ml/min).

On collecte le pic Rt ≅ 16,1 min qui présente un maximum à 304 nm et un rapport $A_{304}/A_{241} \cong 0,79$. On obtient environ 0,03 µmol de composé K-11-dUTP.

La formule du conjugué K-11-dUTP est donnée en figure 2.

<u>EXEMPLE 2</u> : Purification du conjugué K-11-dUTP par échange d'ion

On utilise une colonne C10/20 (Pharmacia Biotech., Uppsala, S) remplie de DEAE Sépharose ® Fast Flow (Pharmacia) équilibrée dans un tampon TEAB 10 mM contenant 10 % de méthanol. On dépose une solution de K-11-dUTP contenant également du cryptate d'europium trisbipyridine fonctionnalisé non conjugué et on élue la colonne (8 ml/min) par 40 ml de tampon A (TEAB 10 mM contenant 10 % de méthanol). On collecte des fractions de 4 ml et on mesure la fluorescence des fractions éluées (λexcitation = 337 nm, λémission = 620 nm). Le cryptate non conjugué est élué dans les fractions 4 et 5, c'est-à-dire peu après le volume mort de la colonne.

On continue l'élution (8 ml/min) par un gradient linéaire de 10 mM TEAB 10 % méthanol (100 ml) à 200 mM TEAB 10 % méthanol (100 ml) et on collecte des fractions de 5 ml. On observe que la fluorescence (620 nm) est concentrée dans les tubes 43 à 44 ce qui indique que le K-11-dUTP est élué à une concentration ≅ 0,16 mM TEAB. Les fractions contenant le K-11-dUTP sont réunies et concentrées.

<u>EXEMPLE 3</u> : Incorporation du conjugué K-11-dUTP au cours de la copie d'un ADN simple brin par une polymérase (analyse par PAGE et autoradiographie)

L'ADN matrice est obtenu par amplification PCR (de l'anglais «Polymerase Chain Reaction ») d'un fragment du gène k-ras (exon I) limité par les amorces suivantes :

Amorce k-ras EX1 Sens S $^{5'}$d(GGC CTG CTG AAA ATG ACT GAA TAT) 3'
Solution stock à 3 $UA_{260}$/ml soit environ 1,2. $10^{-5}$M
Amorce k-ras EX1 Antisens AS $^{5'}$d(TGT TGG ATC ATA TTC GTC CAC AAA ATG)$_{3'}$
Solution stock à 3 $UA_{260}$/ml soit environ 1,2. $10^{-5}$M.

L'amorce AS utilisée ici pour la PCR ci-dessous est biotinylée en son extrémité 5'. Un ADN double-brin biotinylé est synthétisé par PCR en suivant le protocole suivant :

Milieu PCR

25 µl BUAM (CIS bio international)10X
8 µl amorce Antisens-bio
8 µl amorce Sens
10 µl taq polymérase (1,25U/ml)
10 µl dNTP (quatre dNTP naturels 5mM chaque)
100 µl ADN placenta humain (Sigma) 0,01 µg/µl
89 µl eau milli-Q.

Le milieu PCR est réparti en 5 microtubes (5 x 50 µl), les tubes sont placés dans un thermocycleur et soumis à 31 cycles de PCR suivant le protocole de l'exemple 5.
L'ADN double brin issu de la PCR (équivalent à 25 pmoles d'amorce biotine) est incubé en présence de 300 µg de Dynabeads-streptavidine M-280 (Dynal, N).
Après lavage, l'ADN simple brin (SS ADN) est élué par de la soude 0,1 N puis le surnageant est décanté et neutralisé (HCl dilué), puis concentré jusqu'à un volume résiduel de 60 µl.
Pour la suite de la manipulation, on utilise une amorce AS (non biotinylée) telle que définie ci-dessus marquée au $^{32}P$ décrit dans J. Sambrook et al. Molecular cloning a laboratory manuel, 1989.
On prépare des milieux pour la réaction de copie en utilisant :

- K-11-dUTP fraction 1 ( Rt = 16,4 min) obtenue dans l'exemple 1, diluée à 0,25 mM
- dTTP 0,25 mM
- Mélange de 3 désoxynucléotides triphosphates (dATP, dCTP et dGTP) à raison de 0,625 mM chacun
- Taq ADN polymérase 1,25 U/µl
- Tampon Taq (BUAM) 10X (Cis bio international)
- Amorce AS (3 $UA_{260}$/ ml) et amorce AS marquée au $^{32}P$ (0,06 $UA_{260}$/ml).

Parallèlement, on fait une réaction témoin dans laquelle le K-11-dUTP est remplacé par 0,6 µl de dTTP (0,25 mM) de façon à ce que la concentration en dTTP dans le milieu soit la même que pour les trois autres triphosphates.

| | Volume (µl) |
|---|---|
| BUAM 10X | 1 |
| Taq DNA polymérase | 0,2 |
| Amorce S $^{32}P$ | 2 |
| Amorce S | 0,35 |
| 3 Triphosphate | 0,5 |
| K-11-dUTP | 0,6 |
| dTTP | 0,6 |
| SS ADN | 5 |

On vérifie par électrophorèse sur gel de polyacrylamide (PAGE) que la réaction en présence de conjugué K-11-dUTP produit une bande correspondant à une copie de l'ADN sur toute sa longueur et présentant une mobilité proche de la bande obtenue pour la réaction témoin où seuls les quatre nucléotides naturels sont introduits. D'autre part, dans les deux cas, le profil d'électrophorèse ne montre pas d'arrêts de lecture.
La même manipulation a été effectuée en utilisant des rapports dTTP/K-11-dUTP variables, entre autre en utilisant 0,3 µl de K-11-dUTP et 0,9 µl de dTTP (dTTP/K-11-dUTP = 3).
La réaction de copie a également été effectuée en présence de DNA polymérase 1, fragment de Klenow (37°C, 45 min) et donne sensiblement les mêmes résultats.
Ces résultats montrent que le conjugué dUTP-cryptate d'europium trisbipyridine est bien incorporé par une polymérase (taq polymérase ou fragment de Klenow) au cours de la copie de l'ADN simple brin.

EXEMPLE 4 : Incorporation du conjugué K-11-dUTP au cours de la copie d'un ADN simple brin par une polymérase (mise en évidence par transfert d'énergie non radiatif et mesure de fluorescence en temps résolu)

On utilise l'ADN simple brin décrit dans l'exemple 3.
On prépare un microtube dans lequel se déroule la réaction de copie (50 µl), contenant :

- K-11-dUTP : 0,25 mM fraction 1 (Rt = 16,4 min) obtenu dans l'exemple 1
- dTTP : 0,25 mM
- Mélange de 3 désoxynucléotides triphosphates : dATP, dCTP, dGTP à raison de 0,625 mM chacun
- Amorce AS biotinylée (voir exemple 3) : 3 $UA_{260}$/ml
- Taq DNA polymérase : 1,25 U/µl
- Tampon Taq : BUAM 10X

| | Volume (µl) |
|---|---|
| BUAM 10 X | 5 |
| Taq polymérase | 1 |
| Amorce biotinylée | 1,75 |
| Mix 3 dNTP | 2,5 |
| K-11-dUTP | 3 |
| dTTP | 3 |
| SS ADN | 30 |
| eau milli-Q | 3,75 |

Les tubes sont chauffés 2 min à 90°C (dénaturation) puis incubés à 70°C dans un thermocycleur. Des prélèvements (9 µl) sont effectués à 0, 5, 10, 15 et 20 min. Les aliquotes sont placées dans des tubes contenant 2 µl de solution EDTA 0,5 M pH 8.
On obtient l'équivalent de 12,5 pmoles d'amorces biotinylées par 50 µl de réaction, soit 2,5 pmoles pour 10 µl. D'autre part, on a 150 pmoles de K-11-dUTP pour 10 µl.
Les prélèvements sont dilués à raison de 8 µl dans 200 µl de tampon L (phosphate 0,1 M pH 7, NaCl 0,15 M, KF 0,4 M et 0,1 % BSA) puis dilués au 1/10 dans le même tampon.
On pipette 20 µl (équivalent à $2.10^{-14}$ mole de biotine) de prélèvements (dilués comme décrit ci-dessus) dans les puits « essais » d'une microplaque (96-wells flat bottom black low fluorescence microplaque HTRF-96 PACKARD). On ajoute 30 µl de conjugué $SA\text{-}XL_{665}$ (conjugué de streptavidine et d'allophycocyanine chimiquement modifiée, CIS bio international) dilué dans le tampon L (concentration finale $2.10^{-8}$ M) dans chaque puits « essais » ($6,5.10^{-13}$ mole de SA soit 30 équivalent par rapport à la biotine), puis 250 µl de tampon L.
On pipette 20 µl de prélèvement dilués dans les puits « blancs » auxquels on ajoute 280 µl de tampon L.
Après incubation (15 mn à 37°C) on lit immédiatement la fluorescence à 620 nm et à 665 nm sur un appareil Discovery (Microplate HTRF - analyser PACKARD).
On calcule les rapports des intensités de fluorescence Re = E665/E620 pour chaque essai et Ro = B665/B620 pour chaque blanc, puis on calcule la valeur DF = (Re-Ro)/Ro exprimée en pourcentage, les résultats sont reportés dans le tableau 1 ci-dessous.
Dans le tableau, comme dans la suite des exemples, les mesures de fluorescence à 620 nm ou à 665 nm sont exprimées en unités arbitraires de fluorescence qui dépendent de l'appareil utilisé pour la mesure.

Tableau 1

| temps (min) | E665 | E620 | Re = E665/E620 | B665 | B620 | Ro = B665/B620 | DF |
|---|---|---|---|---|---|---|---|
| 0 | 1 590 | 35 770 | 0,044 | 1 615 | 39 423 | 0,041 | 8% |
| 5 | 3 670 | 42 016 | 0,087 | 1 718 | 42 961 | 0,040 | 118% |
| 10 | 4 077 | 40 448 | 0,100 | 1 713 | 42 234 | 0,040 | 149% |
| 15 | 4 185 | 38 670 | 0,108 | 1 633 | 40 089 | 0,040 | 166% |
| 20 | 5 357 | 49 403 | 0,108 | 1 993 | 51 777 | 0,038 | 182% |

En portant la valeur DF en fonction du temps de réaction, on obtient le profil typique d'une cinétique d'incorporation de nucléotide au cours d'une copie enzymatique (Figure 1).

<u>EXEMPLE 5</u> : Incorporation du conjugué K-11-dUTP au cours d'une réaction de PCR (mise en évidence par transfert d'énergie non radiatif et mesure de fluorescence en temps résolu)

1/ PCR :

On amplifie une région de l'exon 1 du gène k-ras limité par les amorces S et AS décrites dans l'exemple 3.
On génère ainsi un produit d'amplification d'une longueur de 117 bp et de séquence (brin sens)

5' d(GGC CTG CTG AAA [1]ATG ACT GAA TAT AAA CTT GTG GTA GTT
[10]GGA GCT GGT GGC GTA GGC AAG AGT <u>GCC TTG [20]ACG ATA CAG C</u>TA ATT
CAG AAT CAT TTT GTG [30] GAC GAA TAT GAT CCA ACA)3'

On choisit une sonde (56 % G + C) dans la partie centrale de la séquence amplifiée (soulignée ci-dessus) une biotine est introduite en son extrémité 5' à l'aide d'un bras N-4-aminohexyl-dC (A. Roget et al., Nucleic Acids Res., 1989, <u>17,</u> 7643-7651) : Sonde CP : biotine-dC .<u>GCC TTG ACG ATA CAG C</u>
Solution stock à 0,3 $UA_{260}$/ml soit environ $1,7.10^{-6}$ M.
On utilise 48 µl de la fraction K-11-dUTP (13 $UA_{304}$/ml) repurifiée dans un tampon TEAAc (voir exemple 1, méthode A) que l'on dilue par 32 µl d'eau milli-Q soit une concentration finale de K-11-dUTP d'environ 0,2 mM.
On utilise un mélange des 4 désoxynucléotides triphosphates naturels respectivement : dATP 5 mM, dCTP 5 mM, dGTP 5 mM et dTTP 3,5 mM.
On prépare le mélange stock PCR suivant :

- 25 µl Tampon BUAM (CIS bio international) 10X
- 10 µl dNTP
- 8 µl d'amorce S
- 8 µl d'amorce AS
- 10 µl de taq ADN polymérase (soit 12,5 U)
- 9 µl eau milli-Q.

Dans des microtubes pour PCR, on prépare les milieux PCR selon le tableau suivant, en utilisant de l'ADN de placenta humain (Sigma) à 0,01 µg/µl.

| | T- | T+ | k1- | k1+ | k2- | k2+ |
|---|---|---|---|---|---|---|
| mélange (µl) | 7 | 7 | 7 | 7 | 7 | 7 |
| dTTP(µl) | 1,5 | 1,5 | 0 | 0 | 0 | 0 |
| K-11-dUTP (µl) | 0 | 0 | 1 | 1 | 2 | 2 |
| DNA(µl) | 0 | 10 | 0 | 10 | 0 | 10 |
| Eau milli-Q (µl) | 16,5 | 6,5 | 17 | 7 | 16 | 6 |
| [K-11-dUTP] (µM) | 0 | 0 | 8 | 8 | 16 | 16 |
| [dTTP](µM) | 200 | 200 | 140 | 140 | 140 | 140 |
| [K-11-dUTP]/[dTTP) | - | - | 0,06 | 0,06 | 0,12 | 0,12 |

On effectue la réaction PCR en utilisant le protocole suivant :

1. 5 min / 95°C
2.1 1 min / 94°C (dénaturation)
2.2 1 min / 60°C (circularisation)
2.3 1 min / 70°C (élongation)

(31 cycles)
3.1 8 min / 70°C (élongation finale)

On contrôle les réactions par électrophorèse sur gel d'agarose. Seuls des tubes contenant de l'ADN (T+, K1+ et K2+) présentent une bande de longueur attendue (par comparaison avec la bande 124 bp du marqueur VIII Boehringer).

2) Mesure du transfert d'énergie en temps résolu :

Le principe de la mesure consiste à hybrider la sonde CP biotinylée sur le fragment d'ADN amplifié, puis de mettre l'hybride en présence du conjuguéµ SA-XL665 (défini dans l'exemple 4). L'incorporation de bases marquées au cryptate d'europium (donneur) se traduit par un transfert d'énergie non radiatif sur XL665 (accepteur) lorsque le donneur est excité vers 337 nm.

-Dans ce cas, l'accepteur émet de la fluorescence à 665 nm avec une durée de vie longue, ce qui permet de différencier ce signal de la fluorescence propre de l'accepteur qui présente une durée de vie courte.

On mesure en temps résolu à la fluorescence à 620 nm et à 665 nm en présence de l'accepteur ("essai" E620 et E665) et en l'absence de l'accepteur ("blanc" B620 et B665), puis on calcule le rapport Re = E665 / E620 et Ro = B665 / B620. Ensuite on calcule la valeur DF = (Re-Ro) / Ro exprimée en pourcentage une augmentation de valeur DF montre la présence d'un transfert d'énergie et donc l'incorporation du cryptate dans l'ADN amplifié.

On utilise les milieux K1-, K1+, K2- et K2+ provenant des réactions PCR. Dans des microtubes, on dépose 2 µl de chaque milieu PCR auxquels on ajoute 10 µl de sonde CP (0,03 UA260 / ml) et 13 µl de tampon BUAM (2X), on porte les tubes scellés 10 mn à 94°C puis 20 mn à 50°C à l'aide d'un thermocycleur. On dilue 5 µl de milieu d'hybridation dans 200 µl de tampon L et on pipette 40 µl de cette dilution dans les puits "Essais" d'une plaque de microtitration et 40 µl dans les puits "blanc" (96 well flat bottom microplate HTRF, Packard).

On ajoute 40 µl de SA-XL665 à $3.10^{-8}$M (CIS bio international) aux puits "Essais" et 200 µl de tampon L. On ajoute 240 µl de tampon L aux puits "Blanc".

On incube 15 mn à 37°C et on effectue immédiatement la mesure de fluorescence sur un appareil Discovery (Packard).

Les résultats sont rapportés dans le tableau 2 ci-après.

Tableau 2

| tube | E665 | E620 | Re | B665 | B620 | Ro | DF |
|---|---|---|---|---|---|---|---|
| K1- | 889 | 16053 | 0,0554 | 847 | 16825 | 0,0503 | 10% |
| K1+ | 2406 | 28392 | 0,0847 | 1366 | 32494 | 0,0420 | 102% |
| K2- | 1320 | 29015 | 0,0455 | 1273 | 31452 | 0,0405 | 12% |
| K2+ | 3304 | 36615 | 0,0902 | 1491 | 36278 | 0,0411 | 120% |

Les résultats montrent que le conjugué K-11-dUTP est incorporé au cours d'une réaction de PCR. De plus, la présence des molécules de cryptate liées à l'ADN amplifié peut être mise en évidence par l'hybridation d'une sonde spécifique de cet ADN amplifié et révélée par un transfert d'énergie non radiatif entre le cryptate et un accepteur lié à la sonde hybridée.

EXEMPLE 6: Synthèse de la désoxyuridine marquee au cryptate d'europium trisbipyridine (K-4-dUTP).

Dans ce composé, le chiffre 4 indique le nombre total d'atomes du bras qui relie la structure cryptate au nucleotide (ici la liaison se fait en position 5 de la pyrimidine).

Le nucléoside-triphosphate utilisé est le 5-(3-aminoallyl)-2'-désoxyuridine-5'-triphosphate (AA-dUTP) préparé suivant un procédé de la littérature (Langer et al. Proc. Natl. Acad. Sci. U.S.A.(1981), 78, 6633-6637). Le composé est purifié sur DEAE-Sepharose ® (Pharmacia) dans un gradient d'hydrogénocarbonate de triéthylammonium (10mM à 300mM).

On dilue 60 µl d'une solution d'AA-dUTP à 5,4 µmole/ ml (soit 0,3 µmole) à 240 µl d'hydrogénocarbonate de triéthylammonium (TEAB) 0,1 M pH 7,8 et on ajoute 300 µl d'une solution de cryptate [TBP-(Eu3+)] activé à 4 mg/ml dans l'acétonitrile, soit 0,85 µmol (environ 3 équivalents). Le cryptate [TBP-(Eu3+)] activé est préparé extemporanément comme décrit dans l'exemple 1, méthode A.

Après 35 min sous agitation on ajoute 15 µl de TEAB 1M pH 8,5, on concentre de moitié puis on injecte le

mélange sur une colonne Superdex 30 ® HR 10/30 (Pharmacia) en éluant par du TEAB 25mM pH9 contenant 10% d'acétonitrile (débit 1ml/min).

On collecte le composé de Rt ≅ 16,3 mn ; cette fraction dénommée Fraction 1 est concentrée sous vide (speed-vac) jusqu'à un volume de 200 µl puis injectée sur la même colonne éluée par un tampon triéthylammonium acétate 25mM pH7 contenant 5% d'acétonitrile. La fraction correspondant au seul pic du chromatograme est collectée (16 min <Rt< 19 min) et concentrée sous vide (speed-vac) on obtient 260µl d'une solution de K-4-dUTP dénommée Fraction 2 contenant 6,0 $UA_{303}$. En estimant un $\varepsilon_{303} \cong 35\,000$, on estime la concentration en K-4-dUTP à environ 0,65 mM.

Le composé est analysé en spectrométrie de masse (électrospray mode négatif):

$$(M\text{-}4H)^- = 1315.5\ (C_{50}H_{48}EuN_{11}O_{17}P_3\ \text{Calculé: } 1319\ )$$

Le spectre UV dans l'eau présente un maximum à 243 nm caractéristique du nucléoside (λmax = 289 nm ε = 7100, λmax = 240 nm ε = 10700 ) et un maximum à 303 nm proche du λmax de 305 nm (ε = 27000) caractéristique du cryptate d'Europium. On observe un rapport $A_{303}/A_{240} \cong 0{,}82$ compatible avec la structure proposée).

La formule du conjugué K-4-dUTP est donné en figure 2.

<u>EXEMPLE 7</u>: Synthèse de l'uridine marquée au cryptate d'europium trisbipyridine (K-11-UTP).

Dans ce composé, le chiffre 11 indique le nombre total d'atomes du bras espaceur qui relie la structure cryptate au nucleotide (ici la liaison se fait en position 5 de la pyrimidine).

Le nucléoside-triphosphate utilisé est le 6-aminocaproyl-[5-(3-aminoallyl)-Uridine-5'-triphosphate] (AH-UTP) préparé comme indiqué dans l'exemple 1. Le composé est purifié sur DEAE-Sepharose® (Pharmacia) dans un gradient linéaire d'hydrogénocarbonate de triéthylammonium (25mM à 300mM).

On utilise 400 µl d'une solution d'AH-dUTP à 1,1 µmole/ ml (soit 0,44µmole) dans de l'hydrogénocarbonate de triéthylammonium (TEAB) 0,1 M pH 7,8 et on ajoute 360 µl d'une solution de de cryptate (TBP-(Eu3+)] activé (3mg/ ml dans l'acétonitrile). Le cryptate [TBP-(Eu3+)] activé est préparé extemporanément comme décrit dans l'exemple 1, méthode A.

Après 45 min sous agitation on ajoute 40µl de TEAB 1M pH 8,5 puis on injecte le mélange sur une colonne Superdex peptide 30 ® HR 10/30 (Pharmacia) en éluant par du TEAB 50 mM pH 7 contenant 10% d'acétonitrile (débit 1ml/mn).

On collecte le composé de Rt ≅ 15,4 mn, cette fraction dénommée Fraction 1 est concentrée sous vide (speed-vac) j'usqu'à un volume de 200 µl qui contient ≅ 10 $UA_{304}$ nm. En estimant un $\varepsilon_{304} \cong 35\,000$, on estime la concentration en K-11-UTP à environ 0,3 mM.

Cette fraction 1 est injectée sur la même colonne éluée par un tampon triéthylammonium acétate 25mM pH7 contenant 5% d'acétonitrile. La fraction correspondant au seul pic du chromatograme est collectée (Rt ≡ 16,5 min) et concentrée sous vide (speed-vac) on obtient 202 µl d'une solution de K-11-UTP dénommée fraction 2 contenant 7,5 $UA_{304}$ nm.

Le composé est analysé en spectrométrie de masse (électrospray mode négatif):

$$(M\text{-}4H)^- = 1444.3\ (C_{56}H_{58}EuN_{12}O_{19}P_3\ \text{Calculé : } 1448.0).$$

Le spectre UV dans l'eau présente un maximum à 241 nm caractéristique du nucléoside (λmax = 289 nm ε = 7 100 , λmax = 240 nm ε = 10700) et un maximum à 303 nm proche du λmax de 305 nm (ε = 27 000) caractéristique du cryptate d'Europium. On observe un rapport $A_{304}/A_{241} \cong 0{,}80$ compatible avec la structure proposée).

Le composé est analysé par FPLC (colonne mono-Q, Pharmacia). Tampon A: Acetate de sodium 20mM pH 5,2 contenant 10% d'acétonitrile. Tampon B: Acetate de sodium 20mM pH 5,0 et LiCl 1M contenant 10% d'acétonitrile. Gradient linéaire de 0 à 30 % B en 25mn. Débit 1ml/mn. Le K-11-UTP présente un Rt = 10,7 min.

La formule du conjugué K-11-UTP est donnée en Figure 3.

<u>EXEMPLE 8</u> : Mesures comparées des durées de vie des conjugués dUTP-et UTP-cryptate d'europium trisbipyridine et du cryptate seul

On utilise comme cryptate de référence le cryptate d'Europium [(bis-bpy)(bpy-di(amidoéthylèneamine)] décrit dans l'exemple 4 de la demande EP 0 321 353 (ci-après dénommé $KNH_2$).

On prépare une solution mère de $KNH_2$ dans l'eau et on détermine sa concentration par mesure de la densité

optique à 306 nm en prenant un $\varepsilon_{306} \cong 30000$. La solution mère ($6,7 \times 10^{-4}$ M) est diluée au 1/100$^{éme}$ dans du tampon Tris-HCl 0,1M pH 9; 100 µl de cette dilution intermédiaire sont ensuite bdilués dans 600 µl du même tampon. Parallèlement, on prépare une solution mère de K-11-dUTP, K-4-dUTP et K-11-UTP (préparés respectivement selon les exemples 1, méthode A ; 6 et 7) dans l'eau , dont la concentration est estimée à $3 \times 10^{-5}$ M par mesure de la densité optique à 304 nm ($\varepsilon_{304} \cong 35000$). Chaque solution mère est diluée à 50µl dans 1 ml de tampon Tris-HCl 0,1M pH 7,4 ou dans du tampon Tris-HCl 0,1M pH 9.

On mesure les valeurs de la durée de vie d'émission de l'Europium ($\tau$ en ms) en utilisant un spectrofluorimètre en temps résolu de type LS50 (Perkin-Elmer) et des cuves Helma 5 mm x 5 mm.

Les résultats sont rapportés dans le tableau 3 ci-dessous :

Tableau 3

| Composé | Durée de vie $\tau$ (ms) dans un tampon Tris 0,1 M pH 7,4 | Durée de vie $\tau$ (ms) dans un tampon Tris 0,1 M pH 9 |
|---|---|---|
| K-11-dUTP | 0,587 | 0,596 |
| K-4-dUTP | 0,621 | 0,651 |
| K-11-UTP | 0,636 | 0,640 |
| $KNH_2$ (Référence) | 0,368 | 0,265 |

Ces résultats montrent que le couplage du nucléotide sur la molécule de cryptate entraîne une augmentation de la durée de vie de l'Europium et de nouvelles caractéristiques de fluorescence pour le conjugué cryptate- nucléotide par rapport au cryptate de référence.

<u>EXEMPLE 9</u> : Incorporation de K-11-dUTP au cours de l'élongation d'un oligonucléotide par la Terminale Nucléotidyl Transférase (Mise en évidence par transfert d'énergie non radiatif et mesure de fluorescence en temps résolu) :

On prépare un microtube dans lequel se déroule la réaction d'élongation en utilisant :

- 25 µl de tampon Tris-acétate 0,2M pH 7,2
- 4 µl d'une solution aqueuse de chlorure de cobalt 25 mM
- 5 µl d'une solution d'un oligonucléotide (composition $A_2C_3G_7T_3$ biotinylé en 5'). 2,4µM dans $H_2O$
- 2µl de K-11-dUTP (fraction 2, préparé selon l'exemple 7) à une concentration de 0,04 mM
- 8 µl de dTTP: 0,04 mM
- 5 µl d'eau
- 1 µl de Terminal Deoxynucleotidyl Transferase (TnT, EC 2.7.7.31) (Sigma, 35 U/µl).

On prélève 2 µl de mélange réactionnel qu'on ajoute à 4 µl d'EDTA 60 mM (pour obtenir un témoin à $t_0$ = 0 min) et on incube le reste à 37°C pour faire une étude cinétique. Après 5, 10, 20, 40, 60 et 80 min de réaction à 37°C, on prélève pour chaque temps 2 µl de mélange réactionnel qu'on ajoute à 4 µl d'EDTA 60 mM pour arrêter la réaction. On obtient ainsi les fractions $t_5$, $t_{10}$ etc. Ces fractions $t_0$, $t_5$, $t_{10}$ sont diluées par 250 µl de tampon L (phosphate 0,1M pH 7, NaCl 0,15M, KF 0,4M et 0,1% BSA).

On pipette 50 µl (équivalent à $2,3.10^{-12}$ mole de biotine) de prélèvements dilués comme décrit ci-dessus dans les puits "essais" (dénommés $E_0$, $E_5$, $E_{10}$ etc.) d'une microplaque (96-wells flat bottom black low fluorescence microplaque HTRF-96 PACKARD). On ajoute 50 µl de conjugué SA-$XL_{665}$ (CIS bio international) dilué dans le tampon L ($1,5.10^{-8}$ M), dans chaque puit "essais", puis 150 µl de tampon L.

On réalise un "blanc" à partir d'un mélange réactionnel décrit ci-dessus dans lequel l'enzyme n'a pas été ajoutée mais remplacé par 1µl d'eau, on prélève 2µl de ce mélange et on lui fait subir les traitements et dilutions décrits ci-dessus. On pipette 50µl de ce prélèvement dilué dans les puits dénommés "blancs" auxquels on ajoute 50 µl de conjugué SA-$XL_{665}$ et 150 µl de tampon L.

Après incubation (15 min à 37°C), on lit la fluorescence à 620 nm et à 665 nm sur un appareil DISCOVERY (Microplate HTRF-analyser PACKARD).

On calcule les rapports des intensités de fluorescence Re=E665/E620 pour chaque essai et Ro=B665/B620 pour chaque blanc, puis on calcule la valeur DF = (Re-Ro)/Ro exprimée en pourcentage, les résultats sont reportés dans le tableau 4 ci-dessous.

Tableau 4

| Temps min) | 0 | 5 | 10 | 20 | 40 | 60 | 80 |
|---|---|---|---|---|---|---|---|
| F665 | 602 | 1585 | 2186 | 3610 | 5347 | 8204 | 5542 |
| F620 | 9559 | 10757 | 9921 | 11023 | 10754 | 10589 | 8971 |
| Re=F665/F620 | 0,063 | 0,147 | 0,22 | 0,327 | 0,497 | 0,77 | 0,618 |
| DF | 0 | 129 | 242 | 409 | 673 | 1104 | 865 |

Le blanc présente un rapport $B_{665}/B_{620}$ = 0,064 . Pour $t_0$ on observe un rapport $E_{665}$ / $E_{620}$ = 0,063, le transfert étant de DF = 100x (Re-$R_0$)/$R_0$ = 100 (0,063-0,064) / 0,064 soit un transfert pratiquement nul. En calculant ainsi le DF pour chaque temps, on observe que le transfert d'énergie exprimé par la valeur DF augmente au cours du temps, ce qui montre que le K-11-dUTP est incorporé dans la chaine oligonucléotidique par la Terminal Deoxynucleotidyl Transferase.

EXEMPLE 10: Propriétés photophysiques d'un oligonucléotide dans lequel sont incorporés des molécules de cryptate :

On effectue une réaction d'incorporation de K-11-dUTP par la TnT suivant l'exemple 9 en multipliant toutes les quantités par 3. On vérifie que l'incorporation de K-11-dUTP suit la même cinétique en réalisant la mesure de DF en fonction du temps. Après 80mn de réaction, on l'arrête par 12 µl de solution d'EDTA 400 mM. On prélève 105 µl de mélange réactionnel et on le dépose sur une colonne NAP5 (Pharmacia) équilibrée dans du tampon phosphate 10 mM pH 7,4.

L'oligonucléotide est élué par 600 µl de tampon. Cette fraction appelée « fraction 1 » (volume d'exclusion) est analysée comme suit : dans les puits d'une microplaque on dépose 50 µl de fraction 1 auquel on ajoute 200 µl de tampon L ce qui constitue le « blanc cryptate ». Dans les puits suivants on dépose 50 µl de fraction 1 et 50 µl de conjugué SA-$XL_{665}$ (CIS bio international) (1,5. $10^{-8}$ M dans du tampon L) et 150 µl de tampon L ce qui constitue le puits « essai ». On mesure la fluorescence à 620 nm et à 665 nm sur un appareil DISCOVERY (Microplate HTRF-analyser PACKARD.

| | F665 | F620 |
|---|---|---|
| Blanc K | 2343 | 67481 |
| Essai | 42982 | 37722 |

On calcule DF = 100 x (Re-$R_0$) / $R_0$ = 100 (1,14-0,0347) / 0,0347 = 3180%, cette fraction contient donc l'oligonucléotide marqué au cryptate.

On observe que les fractions obtenues en continuant l'élution (fractions 2, 3 ...) présentent une valeur de DF faible par rapport à celle de F1 et une fluorescence brute élevée à 620 nm : elles contiennent donc l'excès de K-11-dUTP non incorporé.

En comparant la fluorescence obtenue dans la fraction F1 (qui correspond aux molécules de cryptate incorporées dans la chaîne oligonucléotidique) avec la fluorescence de solutions étalon contenant du cryptate à une concentration connue, on peut estimer que la fraction F1 contient environ $5.10^{-11}$ mol de cryptate, étant donné que la quantité d'oligonucléotide estimée dans la fraction F1 est de $2.10^{-11}$, on observe un nombre n = $5.10^{-11}/2.10^{-11}$ de nucléotide marqué au cryptate par chaîne oligonucléotidique.

Il faut noter que l'incorporation de K-dUTP et de dTTP se fait de façon simultanée et de façon statistique.

Sur l'extrémité 3' de chaque chaîne oligonucléotidique sont donc incorporés un nombre variable de nucléotides T et en moyenne, au moins 2 nucléotides K-dU'.

La durée de vie $\tau$ est calculée à partir de la pente à la droite obtenue en traçant Log $E_{620}$ = f(t).

On mesure la durée de vie de l'émission à 620nm pour l'oligonucléotide marqué contenu dans la fraction 1 (tampon phosphate 10 mM pH 7,4) en utilisant un fluorimètre en temps résolu KRYPTOR (CIS bio international) avec une excitation à 337 nm. On obtient une durée de vie $\tau$ = 866 µs. Cette durée de vie est supérieure à la durée de vie du conjugué K-11-dUTP ($\tau$ = 681 µs) mesuré simultanément dans les mêmes conditions.

On observe donc une augmentation de la durée de vie du cryptate incorporé dans une chaîne oligonucléotidique.

EXEMPLE 11: Synthèse de l'adénosine marquée au cryptate d'Europium trisbipyridine (conjugué K-8-ATP).

Dans ce composé, le chiffre 8 indique le nombre total d'atomes du bras espaceur qui relie la structure cryptate au nucléotide (ici la liaison se fait en position 8 de la purine).

Le nucléoside-triphosphate utilisé est le [8-(6-aminohexyl)-Adenosine-5'-triphosphate] (AH-ATP, Sigma). On utilise une solution de 0,1µmol AH-ATP dans 100 µl d'hydrogénocarbonate de triéthylammonium (TEAB) 0,1 M pH 8 et on ajoute 100 µl d'une solution de cryptate [TBP-(Eu3+)] activé (4 mg/ml dans l'acétonitrile). Le cryptate [TBP-(Eu3+)] activé (NHS/DCC dans l'acétonitrile) est préparé extemporanément comme décrit dans l'exemple 1, méthode A.

Après 35 min sous agitation on ajoute 5 µl de TEAB 1M, on concentre de moitié puis on injecte le mélange sur une colonne Superdex 30 ® HR 10/30 (Pharmacia) en éluant par du TEAB 50 mM pH 8 contenant 10% d'acétonitrile (débit 1ml/min).

On collecte le composé de Rt ≅ 16,7 min, cette fraction dénommée Fraction 1 est concentrée sous vide (speed-vac) jusqu'à un volume de 200 µl puis est injectée sur la même colonne éluée par un tampon triéthylammonium acétate 25mM pH7 contenant 5% d'acétonitrile. La fraction correspondant au seul pic du chromatogramme est collectée (Rt = 17,2 min) et concentrée sous vide (speed-vac).

Le spectre UV dans l'eau présente un maximum à 245 nm, un maximum à 283 nm proche de la λmax caractéristique du nucléoside (λmax= 279, ε = 21 000) et un maximum à 303 nm proche de la λmax de 305 nm (ε =27 000) caractéristique du cryptate d'Europium. On observe un rapport $A_{305}/A_{280} \cong 1$ compatible avec la structure proposée.

La formule du conjugué K-8-ATP est donnée en Figure 3.

EXEMPLE 12 : Incorporation d'un conjugué UTP-cryptate d'europium trisbipyridine (K-11-UTP) au cours d'une réaction de transcription in-vitro (mise en évidence par transfert d'énergie non radiatif et mesure de fluorescence en temps résolu)

On utilise l'incorporation simultanée de K-11-UTP et de Bio-14-CTP (conjugué CTP-Biotine) sur une même molécule d'ARN par transcription in-vitro.

1/ Transcription

La réaction de transcription d'un ADN double brin renfermant un promoteur (ADN plasmidique pSTP18 et pSTP19 contenant le promoteur T7 et linéarisé par *Eco* R1) en ARN est faite à l'aide d'un kit de transcription SP6/T7 (Boehringer-Mannheim). Le transcrit obtenu possède une longueur de 1035 bases.

Le milieu de transcription contient en plus des ribonucléotides à une concentration finale de 0,5mM, 0,2% de K-11-UTP et 30% de bio-14-CTP qui seront incorporés statistiquement au long de la chaîne.

On utilise le K-11-UTP fraction 2 décrit dans l'exemple 7 qui est dilué dans l'eau de façon à obtenir une concentration de 20 µM.

La solution de bio-14-CTP est préparée par dilution dans l'eau d'une solution commerciale 10 mM de Biotine-14-CTP (Gibco-BRL/Life Technologies).

Le milieu de transcription est préparé dans des microtubes pour PCR (0,5ml) suivant le tableau ci-dessous :

| | Volume | Concentration finale | RNTPmarqué / rNTPmarqué+rNTPnaturel |
|---|---|---|---|
| Tampon de transcription (10X) | 2µl | | |
| ADN plasmide (T7) (0,5µg /µl) | 2µl | | |
| ATP (5 mM) | 2µl | 0,5 mM | |
| GTP (5 mM) | 2µl | 0,5 mM | |
| UTP (5 mM) | 2µl | 0,5 mM | |
| K-11-UTP (20µM) | 2µl | 2 µM | 0,4% |
| CTP (3,5 mM) | 2µl | 0,35 mM | |
| Bio-14-CTP (1,5 mM) | 2µl | 0,15 mM | 30 % |
| Inhibiteur de RNase (20 U /µl) | 1µl | | |
| $H_2O$ | 1µl | | |
| T7 RNA polymerase | 2µl | | |

(suite)

| | Volume | Concentration finale | RNTPmarqué / rNTPmarqué+rNTPnaturel |
|---|---|---|---|
| Volume total | 20µl | | |

Après ajout de l'enzyme, on prélève 2 µl de milieu réactionnel qui sont immédiatement dilués par 38 µl d'une solution EDTA 50 mM pH 8, cette solution est ensuite diluée en prélevant 19 µl qui sont dilués par 114 µl de tampon L. Cette solution servira à constituer un témoin de bruit de fond à to = 0 min.

Le mélange réactionnel est placé à 37°C dans un thermocycleur. Après des temps de réaction déterminés (60 min, 90 min et 120 min), on prélève 2 µl de mélange réactionnel qui sont dilués comme précédemment par 38 µl d'EDTA 50 mM pH 8. Ces solutions sont ensuite diluées en prélevant 19 µl qui sont dilués par 114 µl de tampon L.

On dépose 50 µl de chaque solution diluée correspondant aux temps 0 min, 60 min, 90 min et 120 min dans les puits d'une microplaque noire (96 well flat bottom microplate HTRF, Packard).

On ajoute 50µl d'une solution de SA-XL665 (CIS bio international) à une concentration de $6,25.10^{-7}$ M dans du tampon L. On ajoute ensuite 100 µl de tampon L.

2/ Mesure du transfert d'énergie en temps résolu

Après incubation (15 min à température ambiante) on effectue une mesure de fluorescence à 620 nm et à 665 nm sur un appareil DISCOVERY (Packard).

Pour chaque temps de réaction considéré, on évalue le transfert d'énergie en calculant le rapport Re = F665/F620. Le rapport Ro = F665/F620 pour le temps $t_0$ permet d'évaluer le niveau du bruit de fond, la même mesure faite sur une dilution de 2 µl d'un mélange réactionnel de transcription dans lequel l'enzyme a été remplacé par un volume équivalent d'eau donne le même niveau de bruit de fond.

Le transfert d'énergie est calculé par la formule DF = (Re-Ro)/Ro pour chaque temps. Les résultats sont reportés dans le tableau 5 ci-dessous.

Tableau 5

| Temps (mn) | F665 | F620 | Re = F665/F620 | DF = (Re-Ro)/Ro (%) |
|---|---|---|---|---|
| 0 | 2108 | 14672 | 0,143 | 0 |
| 60 | 6329 | 18755 | 0,337 | 135 |
| 90 | 6672 | 17026 | 0,392 | 174 |
| 120 | 7386 | 18179 | 0,406 | 184 |

On observe que la valeur DF augmente, traduisant une augmentation du transfert d'énergie provenant de l'incorporation du K-11-UTP et du bio-CTP dans l'ARN transcrit.

La réaction de transcription décrite ci-dessus est contrôlée par électrophorèse sur gel d'agarose (3%),

On observe une bande de taille élevée montrant l'intégrité du fragment d'ARN produit, cette bande est caractérisée par une migration identique à la bande produite à partir d'une transcription faite à partir des rNTP naturels seuls.

La courbe des valeurs de DF en fonction du temps de réaction donnée en figure 4 montre le profil typique d'une cinétique d'incorporation de nucléotide au cours d'une transcription enzymatique. Ces résultats montrent que le conjugué K-11-UTP est incorporé efficacement par une RNA polymérase.

EXEMPLE 13 : Incorporation d'un conjugué K-11-dUTP au cours d'une réaction de transcription in-vitro (mise en évidence par transfert d'énergie non radiatif et mesure de fluorescence en temps résolu après hybridation d'une sonde-accepteur) :

La réaction de transcription d'un ADN double brin en ARN est faite à l'aide d'un kit de transcription (Gibco BRL). L'ADN double brin renfermant le promoteur T7 est lui-même obtenu par une PCR réalisée avec un couple d'amorces dont une des amorces contient la séquence du promoteur de la T7 RNA polymérase (EC 2.7.7.6).

Le transcrit obtenu possède une longueur théorique de 115 bases.

Le principe général de la transcription par une RNA polymérase est décrit p. 5.58 et 5.59 de «Molecular Cloning, A Laboratory Manual » 2nd edition, J. Sambrook, E.F. Fritsch et T. Maniatis CSH Press 1989.

On peut également utiliser une autre RNA polymérase tel que SP6 ou T3 RNA polymérase : dans ce cas, la

séquence de l'amorce PCR concernée sera modifiée pour incorporer le promoteur spécifique de la RNA polymérase considérée.

L'incorporation d'un promoteur est décrit dans le § 13.5 et § 23.2 dans «PCR : Clinical Diagnostics and Research » A; Rolfs et al. Spinger-Verlag (1992) ainsi que dans D.Y. Kwoth et al. Proc. Natl. Acad. Sci. USA, (1989), 86, 1173-1177.

1/ Obtention de l'ADN double brin renfermant le promoteur de la RNA polymérase :

On utilise un protocole analogue à celui décrit dans l'exemple 5, mais en utilisant uniquement des désoxynucléotides afin de générer un produit PCR primaire de 117 bp.

On effectue une deuxième PCR (PCR secondaire) en utilisant comme ADN cible (DNA) 2 µl d'une dilution au 1/100$^{éme}$ du produit de la PCR primaire. Pour cette PCR secondaire, on remplace l'amorce k-ras EX1 Antisens par l'amorce suivante nommée T7-AS de séquence

5' d(*TAA TAC GAC TCA CTA TAG GGG* TGG ATC ATA TTC GTC CAC AAA ATG) 3'.

La partie de cette séquence écrite en italique correspond à la séquence du promoteur de la T7 RNA polymerase.

L'amorce k-ras EX1 Sens possède la séquence suivante :

5' d(CTG.CTG.AAA.ATG.ACT.GAA.TAT) 3'.

A l'issu de cette PCR secondaire, on obtient un ADN double brin d'une longueur théorique de 132 pb et de séquence suivante (brin sens) :

5' d(CTG.CTG.AAA.ATG.ACT.GAA.TAT.AAA.CTT.GTG.GTA.GTT.GGA.GCT.
GGT.GGC.GTA.GGC.AAG.AGT.GCC.TTG.ACG.ATA.CAG.CTA.ATT.CAG.AAT.CAT.
TTT.GTG.GAC.GAA.TAT.GAT.CCA.C*CC.CTA.TAG.TGA.GTC.GTA.TTA*) 3'.

La partie en italique représente la séquence T7 (brin sens) et la partie soulignée représente la séquence correspondant à la sonde lors de l'étape d'hybridation.

La transcription va produire la séquence ARN suivante (séquence homologue au brin antisens du fragment d'ADN transcrit) dans laquelle la partie soulignée correspond à la séquence qui est reconnue par la sonde biotinylée :

5' pppG.UGG.AUC.AUA.UUC.GUC.CAC.AAA.AUG.AUU.CUG.AAU.UAG.
CUG.UAU.CGU.CAA.GGC.ACU.CUU.GCC.UAC.GCC.ACC.AGC.UCC.AAC.UAC.CA
C.AAG.UUU.AUA.UUC.AGU.CAU.UUU.CAG.CAG.GCC 3'.

La sonde RAS12N utilisée est biotinylée en 5' et possède la séquence suivante :

Biotine-5' d(GTT.GGA.GCT.GGT.GGC.GTA.GG) 3'.

2/ Transcription :

Le milieu de transcription contient, en plus des ribonucléotides naturels à une concentration finale de 0,5 mM, 2 % de K-11-UTP qui sera incorporé statistiquement au long de la chaine d'ARN.

On utilise le K-11-UTP fraction 2 décrit dans l'exemple 7 qui est dilué dans l'eau de façon à obtenir une concentration de 100 µM.

Le milieu de transcription est préparé dans des microtubes pour PCR (0,5 ml) suivant le tableau ci-dessous :

| | Volume | Concentration finale | K-11-UTP / (K-11-UTP+UTP) |
|---|---|---|---|
| Tampon de transcription (5X) | 10µl | | |
| ADN PCR positive (T7) (0,5µg/µl) | 25µl | | |
| DTT 0,1M | 2µl | | |
| Mix rNTPs (5 mM chaque) | 5µl | 0,5 mM | |
| K-11-UTP (100µM) | 5µl | 10 µM | 2% |
| Inhibiteur de RNase (20 U /µl) | 2µl | | |
| $H_2O$ | 0,5µl | | |
| T7 RNA polymerase (50 U /µl) | 0,5µl | | |
| Volume total | 50µl | | |

Le mélange réactionnel est placé dans un thermocycleur à 40°C pendant 120 min, puis on arrête la réaction par 4 µl d'EDTA 0,2 M pH 8.

Dans un microtube pour PCR, on prélève 2 µl du milieu réactionnel et on le dilue par 10 µl de sonde RAS12N (0,03 $UA_{260}$/ml dans $H_2O$) puis on ajoute 13 µl de tampon d'hybridation (tampon phosphate 100 mM pH 7,4 / BSA 0,1 % NaCl 1M). On porte 10 min à 70°C puis on hybride 20 min à 45°C dans un thermocycleur.

5,5 µl du milieu d'hybridation ci-dessus sont dilués dans du tampon L (qsp 200 µl) pour donner le milieu d'hybridation dilué $HT_{pos}$.

On constitue un blanc en effectuant une transcription selon le protocole ci-dessus mais en remplaçant l'ADN de PCR positive par un volume équivalent de milieu PCR provenant d'une réaction de PCR négative qui ne contient pas l'ADN cible.

Le milieu de transcription négative (2 ul) est arrêté, puis on effectue une hybridation comme décrit ci-dessus et on dilue dans du tampon L. On obtient ainsi le milieu d'hybridation dilué $HT_{neg}$.

3/ Mesure du transfert d'énergie en temps résolu

L'incorporation du cryptate dans la chaîne d'ARN est mise en évidence par l'hybridation d'une sonde complémentaire à la séquence d'ARN, cette sonde étant marquée avec un conjugué SA-XL665 (CIS bio international) par l'intermédiaire du couple streptavidine-biotine.

Dans les puits d'une microplaque noire (96 well flat bottom microplate HTRF, Packard), on dépose 50 µl de milieu $HT_{pos}$ et 50 µl de conjugué SA-XL665 (CIS bio international) dilué à $1{,}5.10^{-10}$M dans du tampon L puis on ajoute 100 µl de tampon L. Ces puits vont permettre la mesure du transfert d'énergie par le calcul de Re = E665/E620 détaillé dans l'exemple 5.

Dans les puits voisins, on dépose 50 µl de milieu $HT_{neg}$, on ajoute 50 µl de conjugué SA-XL665 et 100 µl de tampon L comme décrit ci-dessus. Ces puits constituent une référence pour évaluer le bruit de fond par le calcul de Ro.

On effectue une mesure de fluorescence à 620 nm et à 665 nm sur un appareil DISCOVERY (Packard).

Le transfert d'énergie est calculé par la formule suivante DF = (Re-Ro)/Ro pour chaque temps, les résultats sont reportés dans le tableau 6 ci-dessous.

Tableau 6

| Milieu | E665 | E620 | Re = E665/E620 | DF (%) |
|---|---|---|---|---|
| $HT_{neg}$ | 738 | 17900 | 0,041 | |
| $HT_{neg}$ | 1714 | 15010 | 0,114 | 178 |

On observe un transfert de 178 % dans le cas où la transcription est faite en présence de l'ADN cible.

## Revendications

1. Conjugué fluorescent de nucléoside ou de nucléotide, **caractérisé en ce qu'**il comprend :

- un ribo- ou désoxyribo-nucléoside ou nucléotide natif ou chimiquement modifié ou conjugué à une ou plusieurs molécules marqueur(s), dont au moins un atome de carbone du cycle ou un atome d'azote exocyclique du cycle purique ou pyrimidique, ou encore un atome de carbone de l'unité pentofuranose est susceptible d'être impliqué dans une liaison avec un marqueur fluorescent, et
- au moins un marqueur fluorescent lié au(x)dit(s) atome(s) constitué par un cryptate de terre rare.

**2.** Conjugué fluorescent de nucléoside ou de nucléotide, **caractérisé en ce qu'**il comprend :

- un ribo- ou désoxyribo-nucléoside ou nucléotide natif ou chimiquement modifié ou conjugué à une ou plusieurs molécules marqueur(s), dont au moins un atome de carbone du cycle ou d'azote exocyclique du cycle purique ou pyrimidique est susceptible d'être impliqué dans une liaison avec un marqueur fluorescent, et
- au moins un marqueur fluorescent lié au(x)dit(s) atome(s) constitué par un cryptate de terre rare.

**3.** Conjugué selon les revendications 1 ou 2, **caractérisé en ce qu'**il s'agit d'un conjugué fluorescent de nucléotide comprenant un ribo-nucléotide choisi parmi AMP, ADP, ATP, GMP, GDP, GTP, CMP, CDP, CTP, UMP, UDP, UTP, TMP, TDP, TTP, 2Me AMP, 2Me ADP, 2Me ATP, 1Me GMP, 1Me GDP, 1Me GTP, 5Me CMP, 5Me CDP, 5Me CTP, 5MeO CMP, 5MeO CDP, 5MeO CTP, 7-déaza-ATP, 7-déaza-GTP ou un désoxyribo-nucléotide choisi parmi les désoxy- ou didésoxyribonucléotides correspondant à ceux-ci.

**4.** Conjugué selon l'une des revendications 1 à 3, **caractérisé en ce que** le ribo- ou désoxyribo-nucléotide est choisi parmi :

- les dérivés de la 2'-désoxy-uridine-5'-triphosphate ou de l'uridine-5'-triphosphate fonctionnalisés en position 5 de la base,
- les dérivés de la 2'-désoxy-cytidine-5'-triphosphate ou de la cytidine-5'-triphosphate fonctionnalisés en position 4 ou 5 de la base,
- les dérivés de la 2'-désoxy-adénosine-5'-triphosphate ou de l'adénosine-5'-triphosphate fonctionnalisés en position 6 ou 8 de la base,
- les dérivés de la 2'-désoxy-guanosine-5'-triphosphate ou de la guanosine-5'-triphosphate fonctionnalisés en position 6 ou 8 de la base,
- les dérivés de la 2'-désoxy-7-déaza-adénosine-5'-triphosphate ou de la 7-déaza-adénosine-5'-triphosphate fonctionnalisés en position 7 de la base, et
- les dérivés de la 2'-désoxy-7-déaza-guanosine-5'-triphosphate ou de la 7-déaza-guanosine-5'-triphosphate fonctionnalisés en position 7 de la base.

**5.** Conjugué selon la revendication 1, **caractérisé en ce qu'**il s'agit d'un conjugué fluorescent de nucléoside dans lequel le ribo- ou désoxyribo-nucléoside est choisi parmi A, G, C, U, T, les désoxy- ou didésoxynucléosides correspondants, leurs analogues chimiquement modifiés.

**6.** Conjugué selon la revendication 5, **caractérisé en ce que** le désoxyribonucléoside est choisi parmi la 3'-azido-3'-désoxythymidine et ses dérivés ; et les analogues 2', 3'-didésoxy de A, T, C, G, U, I.

**7.** Conjugué selon l'une quelconque des revendications 1 à 6, **caractérisé en ce que** le marqueur fluorescent est lié à un groupe fonctionnel introduit ou généré sur la base ou sur l'unité pentofuranose du ribo- ou désoxyribo-nucléoside ou nucleotide, soit directement, soit par l'intermédiaire d'un bras espaceur.

**8.** Conjugué selon l'une quelconque des revendications 1 à 7, **caractérisé en ce que** le marqueur fluorescent est un cryptate de terbium, d'europium, de samarium ou de dysprosium.

**9.** Conjugué selon l'une quelconque des revendications 1 à 8, **caractérisé en ce que** le marqueur fluorescent est un cryptate de terre rare constitué d'au moins un sel de terre rare complexé par un composé macropolycyclique de formule

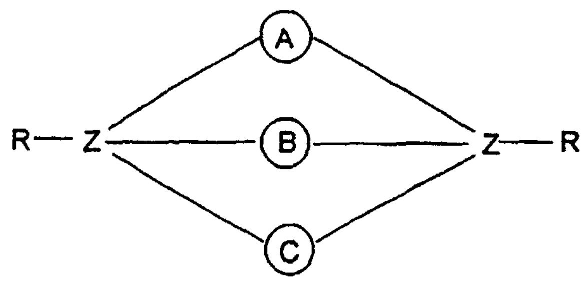

I

dans laquelle Z est un atome ayant 3 ou 4 valences, R est rien ou représente l'hydrogène, le groupe hydroxy, un groupe amino ou un radical hydrocarboné, les radicaux bivalents Ⓐ , Ⓑ et ©, sont indépendamment l'un de l'autre des chaînes hydrocarbonées qui contiennent éventuellement un ou plusieurs hétéroatomes et sont éventuellement interrompues par un hétéromacrocycle, au moins l'un des radicaux Ⓐ , Ⓑ et © comportant de plus au moins un motif moléculaire ou étant essentiellement constitué par un motif moléculaire, ledit motif moléculaire possédant une énergie de triplet supérieure à celle du niveau émissif de l'ion de terre rare complexé.

**10.** Conjugué selon la revendication 9, **caractérisé en ce que** le marqueur fluorescent est un cryptate de terre rare constitué d'au moins un sel de terre rare complexé par un composé macropolycyclique de formule (I) selon la revendication 9, dans laquelle le motif moléculaire est choisi parmi la phénanthroline, l'anthracène, le benzène, le naphtalène, les bi- et ter-phényle, l'azobenzène, l'azopyridine, la pyridine, les bipyridines, les bisquinoléines et les composés de formules ci-après :

$$- C2H4 -X_1-C_6H_4 - X_2 - C_2H_2-$$

$$-C_2H_2-X_1-CH_2-C_6H_4-CH_2-X_2-C_2H_4-$$

$X_1$ et $X_2$ pouvant être identiques ou différents désignent l'oxygène, l'azote ou le soufre,

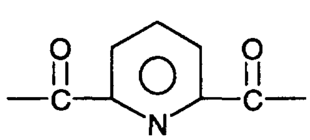

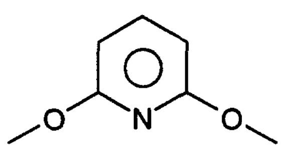

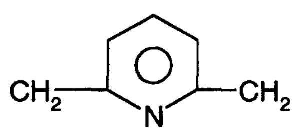

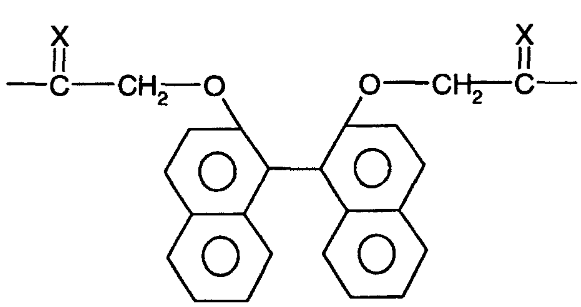

X étant l'oxygène ou l'hydrogène.

**11.** Conjugué selon la revendication 9 ou 10, **caractérisé en ce que** le marqueur fluorescent est un cryptate de terre

rare constitué de l'ion terbium ou europium complexé par l'un des composés macrocycliques ci-après :

(22)phénanthroline ; (22)phénanthroline amide ; (22)anthracène ; (22)anthracène amide ; (22)bi-isoquinoléine ; (22)biphényl-bis-pyridine ; (22)bipyridine ; (22)bi-pyridine amide; les macropolycycles tris-bi-pyridine, trisphénanthroline, phénanthroline-bis-bipyridine, bi-isoquinoléine-bis-bipyridine, bis-bipyridine diphénylbipyridine.

**12.** Conjugué selon la revendication 11, **caractérisé en ce que** le marqueur fluorescent est le cryptate d'europium Eu trisbipyridine.

**13.** Conjugué selon l'une des revendications 1 à 8, **caractérisé en ce que** le marqueur fluorescent est un cryptate de terre rare constitué d'au moins un sel de terre rare complexé par un composé macropolycyclique comprenant un motif moléculaire choisi parmi les bipyrazines, les bipyrimidines et les hétérocycles azotés comportant des groupements N-oxydes.

**14.** Conjugué selon l'une quelconque des revendications 1 à 8, **caractérisé en ce que** le marqueur fluorescent est un cryptate de terre rare constitué d'au moins un sel de terre rare complexé par un composé macropolycyclique répondant à l'une des formules II ou III ci-après :

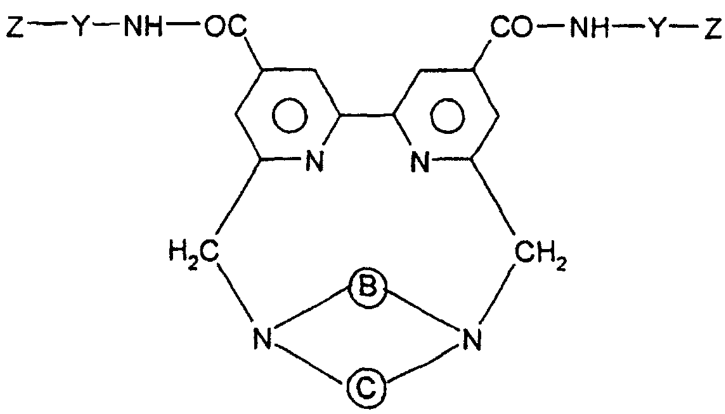

II

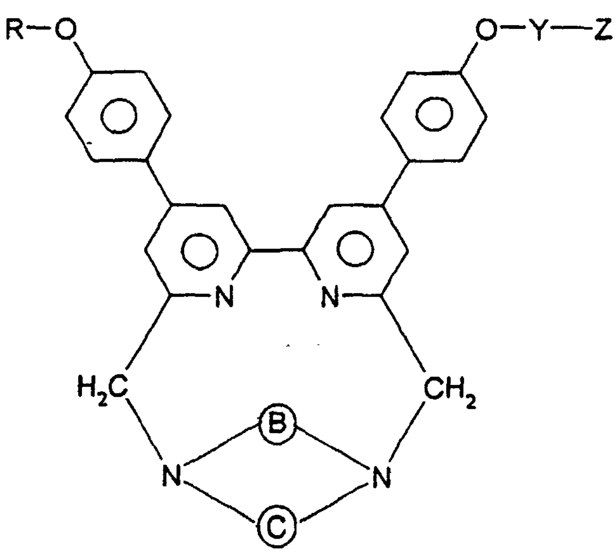

III

dans lesquels :

- le cycle de formule

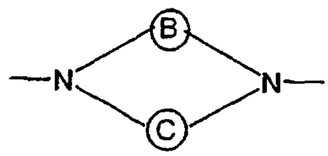

est l'un des cycles suivants :

1) 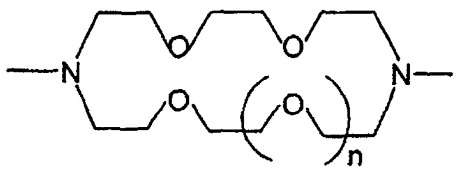

n = 0 ou 1
macrocycle $[N_2O_4]$ ou cycle (22)
macrocycle $[N_2O_3]$ ou cycle (21)

2) 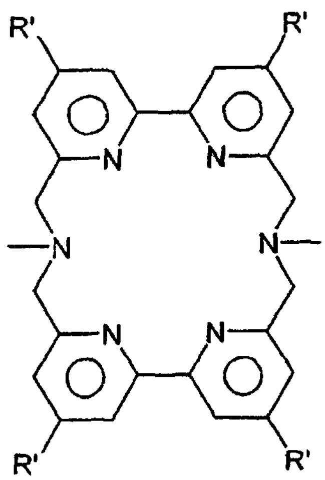

macrocycle bis-bipyridine

- Y est un groupe ou un bras d'espacement qui est constitué par un radical organique bivalent, choisi parmi les groupes alkylène linéaires ou ramifiés en $C_1$ à $C_{20}$ contenant éventuellement une ou plusieurs doubles liaisons et/ou éventuellement contenant par un ou plusieurs hétéroatomes tels que l'oxygène, l'azote, le soufre, le phosphore ou un ou plusieurs groupe(s) carbamoyle ou carboxamido ; parmi les groupes cycloalkylène en $C_5$ à $C_8$ ou parmi les groupes arylène en $C_6$ à $C_{14}$, lesdits groupes alkylène, cycloalkylène ou arylène étant éventuellement substitués par des groupes alkyle, aryle ou sulfonate ;
- Z est un groupe fonctionnel susceptible de se lier de façon covalente avec une substance biologique ;
- R est un groupe méthyle ou représente le groupe -Y-Z ;
- R' est l'hydrogène ou un groupe -COOR" dans lequel R" est un groupe alkyle en $C_1$ à $C_{10}$ et représente de préférence le groupe méthyle, éthyle ou tertiobutyle ou bien R' est un groupe -CO-NH-Y-Z.

**15.** Conjugué selon l'une quelconque des revendications 1 à 14, **caractérisé en ce que** le marqueur fluorescent est lié au ribo- ou au désoxyribonucléoside ou nucléotide par l'intermédiaire d'un bras d'espacement constitué par un radical organique bivalent, choisi parmi les groupes alkylène linéaires ou ramifiés en $C_1$-$C_{20}$, contenant éventuellement une ou plusieurs doubles liaisons ou triples liaisons et/ou contenant éventuellement un ou plusieurs hétéroatomes, tels que l'oxygène, l'azote, le soufre, le phosphore ou un ou plusieurs groupe(s) carbamoyle ou carboxamido; les groupes cycloalkylène en $C_5$-$C_8$ et les groupes arylène en $C_6$-$C_{14}$, lesdits groupes alkylène, cycloalkylène ou arylène étant éventuellement substitués par des groupes alkyle, aryle ou sulfonate.

**16.** Conjugué selon la revendication 15, **caractérisé en ce que** le bras d'espacement est choisi parmi les groupes :

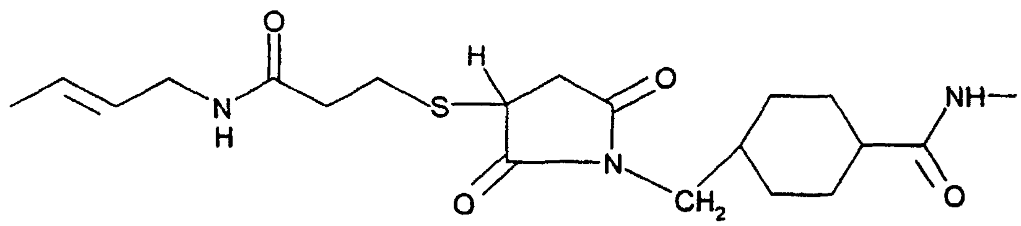

et

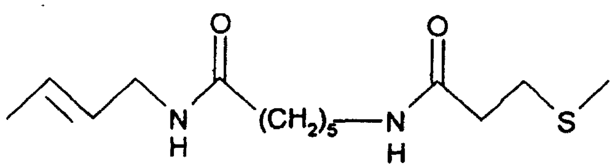

**17.** Conjugué selon l'une des revendications 1 à 15, **caractérisé en ce que** le désoxyribonucléotide est la désoxyuridine, le marqueur fluorescent est le cryptate d'europium Eu trisbipyridine et le bras d'espacement est un groupe 3-aminoallyle.

**18.** Procédé de préparation des conjugués selon l'une quelconque des revendications 1 à 17, **caractérisé en ce qu'**on fait réagir un ribo- ou désoxyribonucléoside ou nucléotide natif ou chimiquement modifié ou conjugué à une ou plusieurs molécules marqueur(s), dont au moins un atome de carbone du cycle ou un atome d'azote exocyclique du cycle purique ou pyrimidique ou encore un atome de carbone de l'unité pentafuronose est susceptible d'être impliqué dans une liaison avec un marqueur fluorescent avec au moins un marqueur fluorescent lié au(x)dit(s) atome(s) constitué par un cryptate de terre rare.

**19.** Utilisation d'un conjugué selon l'une quelconque des revendications 1 à 17 à titre de marqueurs.

**20.** Utilisation d'un conjugué selon l'une quelconque des revendications 1 à 3 et 6 à 16 en tant que nucléotide constitutif dans une réaction de synthèse d'acide nucléique.

**21.** Utilisation d'un conjugué selon l'une quelconque des revendications 1 à 4 et 7 à 16 pour la détection et/ou la localisation de composés contenant au moins une séquence d'acide nucléique.

**22.** Utilisation selon la revendication 20 dans un procédé de mesure de l'activité enzymatique d'une enzyme impliquée dans une réaction de synthèse d'acide nucléique, **caractérisée en ce que** l'on mesure la fluorescence émise directement ou indirectement par ledit conjugué, ladite émission de fluorescence étant corrélée au taux d'incorporation dudit conjugué dans le polynucléotide d'acide nucléique synthétisé.

**23.** Utilisation selon la revendication 22, **caractérisée en ce que** l'activité enzymatique est une activité polymérase à ADN ou à ARN, transcriptase inverse, transférase ou ligase.

**24.** Utilisation selon la revendication 20 dans un procédé de mesure de l'activité enzymatique ayant pour substrat un acide nucléique.

**25.** Polynucléotide comprenant un nombre supérieur à 1 de conjugués selon l'une des revendications 9 à 16.

**Patentansprüche**

**1.** Fluoreszierendes Nucleosid- oder Nucleotid-Konjugat, **dadurch gekennzeichnet, daß** es:

- ein natives oder chemisch modifiziertes oder mit einem oder mehreren Markermolekülen konjugiertes Ribo-

oder Desoxyribonucleosid oder -nucleotid, worin mindestens ein Kohlenstoffatom des Rings, ein exocyclisches Stickstoffatom des Purin- oder Pyrimidinrings oder ein Kohlenstoffatom der Pentofuranoseeinheit mit dem Fluoreszenzmarker eine Bindung eingehen kann, und

- mindestens einen an das Atom bzw. die Atome gebundenen Fluoreszenzmarker, der aus einem Seltenerdmetallkryptat besteht,

enthält.

2. Fluoreszierendes Nucleosid- oder Nucleotid-Konjugat, **dadurch gekennzeichnet, daß** es:

- ein natives oder chemisch modifiziertes oder mit einem oder mehreren Markermolekülen konjugiertes Ribo- oder Desoxyribonucleosid oder -nucleotid, worin mindestens ein Kohlenstoffatom des Rings oder ein exocyclisches Stickstoffatom des Purin- oder Pyrimidinrings mit dem Fluoreszenzmarker eine Bindung eingehen kann, und
- mindestens einen an das Atom bzw. die Atome gebundenen Fluoreszenzmarker, der aus einem Seltenerdmetallkryptat besteht,

enthält.

3. Konjugat nach Anspruch 1 oder 2, **dadurch gekennzeichnet, daß** es sich um ein fluoreszierendes Nucleotid-Konjugat handelt, das ein unter AMP, ADP, ATP, GMP, GDP, GTP, CMP, CDP, CTP, UMP, UDP, UTP, TMP, TDP, TTP, 2Me-AMP, 2Me-ADP, 2Me-ATP, 1Me-GMP, 1Me-GDP, 1Me-GTP, 5Me-CMP, 5Me-CDP, 5Me-CTP, 5MeO-CMP, 5MeO-CDP, 5MeO-CTP, 7-Desaza-ATP oder 7-Desaza-GTP ausgewähltes Ribonucleotid oder ein unter den diesen Ribonucleotiden entsprechenden Desoxy- oder Didesoxyribonucleotiden ausgewähltes Desoxyribonucleotid enthält.

4. Konjugat nach einem der Ansprüche 1 bis 3, **dadurch gekennzeichnet, daß** das Ribo- oder Desoxyribonucleotid unter:

- in 5-Stellung der Base funktionalisierten Derivaten von 2'-Desoxyuridin-5'-triphosphat oder Uridin-5'-triphosphat,
- in 4- oder 5-stellung der Base funktionalisierten Derivaten von 2'-Desoxycytidin-5'-triphosphat oder Cytidin-5'triphosphat,
- in 6- oder 8-Stellung der Base funktionalisierten Derivaten von 2'-Desoxyadenosin-5'-triphosphat oder Adenosin-5'triphosphat,
- in 6- oder 8-Stellung der Base funktionalisierten Derivaten von 2'-Desoxyguanosin-5'-triphosphat oder Guanosin-5'triphosphat,
- in 7-Stellung der Base funktionalisierten Derivaten von 2'-Desoxy-7-desazaadenosin-5'triphosphat oder 7-Desazaadenosin-5'triphosphat und
- in 7-Stellung der Base funktionalisierten Derivaten von 2'-Desoxy-7-desazaguanosin-5'triphosphat oder 7-Desazaguanosin-5'triphosphat

ausgewählt ist.

5. Konjugat nach Anspruch 1, **dadurch gekennzeichnet, daß** es sich um ein fluoreszierendes Nucleosid-Konjugat handelt, bei dem das Ribo- oder Desoxyribonucleosid unter A, G, C, U, T, den entsprechenden Desoxy- oder Didesoxynucleosiden und chemisch modifizierten Analogen davon ausgewählt ist.

6. Konjugat nach Anspruch 5, **dadurch gekennzeichnet, daß** das Desoxyribonucleosid unter 3'-Azido-3'desoxythymidin und Derivaten davon und den 2',3'-Didesoxyanalogen von A, T, C, G, U und I ausgewählt ist.

7. Konjugat nach einem der Ansprüche 1 bis 6, **dadurch gekennzeichnet, daß** der Fluoreszenzmarker entweder direkt oder über einen Spacer an eine an der Base oder der Pentofuranoseeinheit des Ribo- oder Desoxyribonucleosids oder -nucleotids eingeführte oder gebildete funktionelle Gruppe gebunden ist.

8. Konjugat nach einem der Ansprüche 1 bis 7, **dadurch gekennzeichnet, daß** es sich bei dem Fluoreszenzmarker um ein Kryptat des Terbiums, Europiums, Samariums oder Dysprosiums handelt.

**9.** Konjugat nach einem der Ansprüche 1 bis 8, **dadurch gekennzeichnet, daß** es sich bei dem Fluoreszenzmarker um ein Seltenerdmetallkryptat handelt, das aus mindestens einem durch eine makropolycyclische Verbindung der Formel

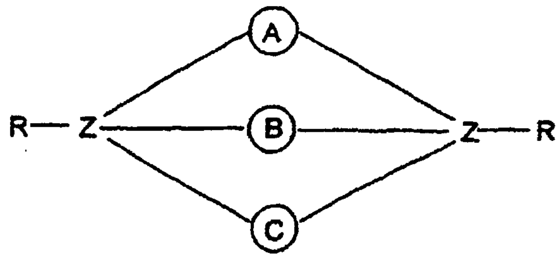

I

worin Z für ein drei- oder vierbindiges Atom steht, R nicht vorhanden ist oder für Wasserstoff, Hydroxyl, eine Aminogruppe oder einen Kohlenwasserstoffrest steht, die zweiwertigen Reste Ⓐ , Ⓑ und © unabhängig voneinander für Kohlenwasserstoffketten, die gegebenenfalls ein oder mehrere Heteroatome enthalten und gegebenenfalls durch einen Heteromakrocyclus unterbrochen sind, stehen, wobei mindestens einer der Reste Ⓐ , Ⓑ und © außerdem mindestens eine Molekülgruppierung enthält oder im wesentlichen aus einer Molekülgruppierung besteht, deren Triplettenergie über der Triplettenergie des Emissionsniveaus des komplexierten Seltenerdmetallions liegt, komplexierten Seltenerdmetallsalz besteht.

**10.** Konjugat nach Anspruch 9, **dadurch gekennzeichnet, daß** es sich bei dem Fluoreszenzmarker um ein Seltenerdmetallkryptat handelt, das aus mindestens einem durch eine makropolycyclische Verbindung der Formel (I) gemäß Anspruch 9 komplexierten Seltenerdmetallsalz besteht, bei dem die Molekülgruppierung unter Phenanthrolin, Anthracen, Benzol, Naphthalin, Bi- und Terphenyl, Azobenzol, Azopyridin, Pyridin, Bipyridinen, Bischinolinen und Verbindungen der nachstehenden Formeln ausgewählt ist:

$$- C_2H_4-X_1-C_6H_4-X_2-C_2H_4-$$

$$- C_2H_4-X_1-CH_2-C_6H_4-CH_2-X_2-C_2H_4- ,$$

wobei $X_1$ und $X_2$ gleich oder verschieden sein können und für Sauerstoff, Stickstoff oder Schwefel stehen,

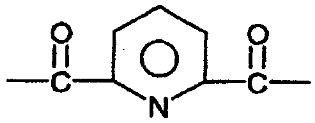

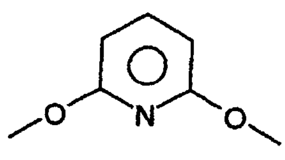

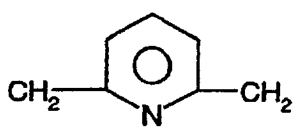

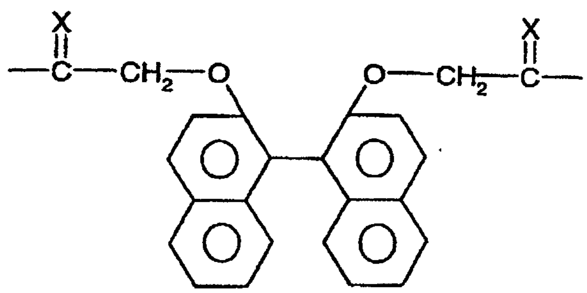

wobei X für Sauerstoff oder Wasserstoff steht.

**11.** Konjugat nach Anspruch 9 oder 10, **dadurch gekennzeichnet, daß** es sich bei dem Fluoreszenzmarker um ein Seltenerdmetallkryptat handelt, das aus einem durch eine der nachstehenden makrocyclischen Verbindungen komplexierten Terbium- oder Europiumion besteht:

(22)Phenanthrolin, (22)Phenanthrolinamid, (22)Anthracen, (22)Anthracenamid, (22)Biisochinolin, (22)Biphenyl-bis-pyridin, (22)Bipyridin, (22)Bi-pyridinamid und Trisbipyridin-, Tris-phenanthrolin-, Phenanthrolinbis-bipyridin-, Bi-isochinolin-bis-bipyridin- und Bis-bipyridin-diphenylbipyridin-Makropolycyclen.

**12.** Konjugat nach Anspruch 11, **dadurch gekennzeichnet, daß** es sich bei dem Fluoreszenzmarker um das Europiumkryptat Eu-trisbipyridin handelt.

**13.** Konjugat nach einem der Ansprüche 1 bis 8, **dadurch gekennzeichnet, daß** es sich bei dem Fluoreszenzmarker um ein Seltenerdmetallkryptat handelt, das aus mindestens einem durch eine makropolycyclische Verbindung mit einer unter Bipyrazinen, Bipyrimidinen und Stickstoffheterocyclen mit N-Oxid-Gruppen ausgewählte Molekülgruppierung komplexierten Seltenerdmetallsalz besteht.

**14.** Konjugat nach einem der Ansprüche 1 bis 8, **dadurch gekennzeichnet, daß** es sich bei dem Fluoreszenzmarker um ein Seltenerdmetallkryptat handelt, das aus mindestens einem durch eine makropolycyclische Verbindung einer der nachstehenden Formeln II oder III komplexierten Seltenerdmetallsalz besteht:

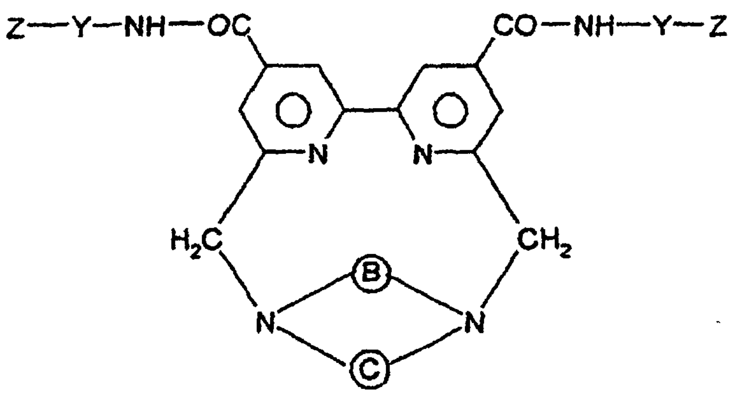

II

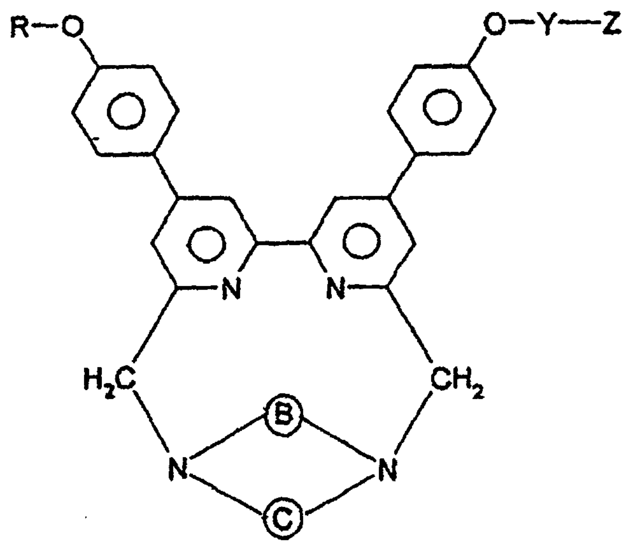

III

worin:

- es sich bei dem Ring der Formel

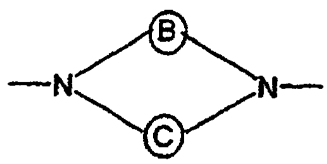

um einen der folgenden Ringe handelt:

1) 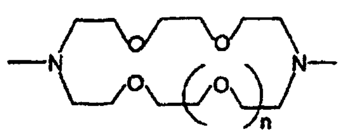

n = 0 oder 1
[$N_2O_4$]-Makrocyclus oder (22)-Ring
[$N_2O_3$]-Makrocyclus oder (21)-Ring

2) 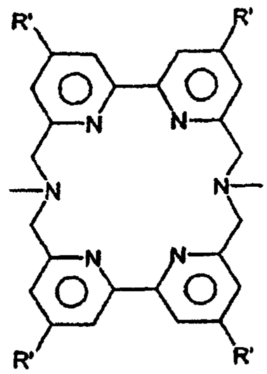 Bis-bipyridin-Makrocyclus

- Y für eine Gruppe oder einen Spacer steht, der aus einem zweiwertigen organischen Rest besteht, der unter linearen oder verzweigten $C_1$bis $C_{20}$-Alkylengruppen, die gegebenenfalls eine oder mehrere Doppelbindungen und/oder gegebenenfalls ein oder mehrere Heteroatome, wie Sauerstoff, Stickstoff, Schwefel und Phosphor, oder eine oder mehrere Carbamoyloder Carbonsäureamidgruppen enthalten können; $C_5$- bis $C_8$-Cycloalkylengruppen oder $C_6$- bis $C_{14}$-Arylengruppen, wobei die Alkylen-, Cycloalkylen- oder Arylengruppen gegebenenfalls durch Alkyl-, Aryl- oder Sulfonatgruppen substituiert sind, ausgewählt ist;
- Z für eine funktionelle Gruppe, die mit einer biologischen Substanz eine kovalente Bindung eingehen kann, steht;
- R für eine Methylgruppe oder die Gruppe -Y-Z steht;
- R' für Wasserstoff oder eine COOR"-Gruppe, worin R" eine $C_1$- bis $C_{10}$-Alkylgruppe und vorzugsweise Methyl, Ethyl oder tert.-Butyl bedeutet, oder eine -CO-NH-Y-Z-Gruppe steht.

**15.** Konjugat nach einem der Ansprüche 1 bis 14, **dadurch gekennzeichnet, daß** der Fluoreszenzmarker über einen Spacer an das Ribo- oder Desoxyribonucleosid oder -nucleotid gebunden ist, welcher aus einem zweiwertigen organischen Rest besteht, der unter linearen oder verzweigten $C_1$-$C_{20}$-Alkylengruppen, die gegebenenfalls eine oder mehrere Doppel- oder Dreifachbindungen und/oder gegebenenfalls ein oder mehrere Heteroatome, wie Sauerstoff, Stickstoff, Schwefel und Phosphor, oder eine oder mehrere Carbamoyl- oder Carbonsäureamidgruppen enthalten können; $C_5$-$C_8$-Cycloalkylengruppen und $C_6$-$C_{14}$-Arylengruppen, wobei die Alkylen-, Cycloalkylen- oder Arylengruppen gegebenenfalls durch Alkyl-, Aryl- oder Sulfonatgruppen substituiert sind, ausgewählt ist.

**16.** Konjugat nach Anspruch 15, **dadurch gekennzeichnet, daß** der Spacer unter den Gruppen:

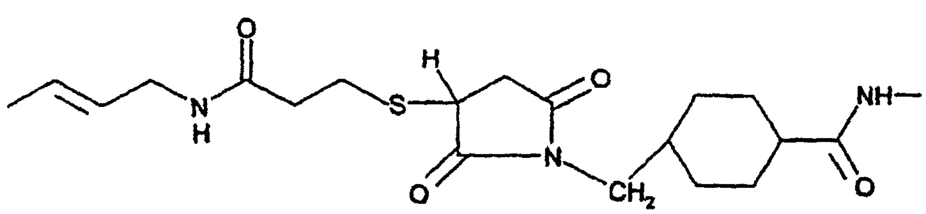

und

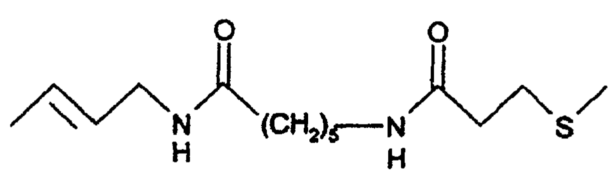

ausgewählt ist.

**17.** Konjugat nach einem der Ansprüche 1 bis 15, **dadurch gekennzeichnet, daß** es sich bei dem Desoxyribonucleotid um Desoxyuridin, bei dem Fluoreszenzmarker um das Europiumkryptat Eu-trisbipyridin und bei dem Spacer um eine 3-Aminoallylgruppe handelt.

**18.** Verfahren zur Herstellung von Konjugaten nach einem der Ansprüche 1 bis 17, **dadurch gekennzeichnet, daß** man ein natives oder chemisch modifiziertes oder mit einem oder mehreren Markermolekülen konjugiertes Ribo- oder Desoxyribonucleosid oder -nucleotid, worin mindestens ein Kohlenstoffatom des Rings, ein exocyclisches Stickstoffatom des Purin- oder Pyrimidinrings oder ein Kohlenstoffatom der Pentofuranoseeinheit mit dem Fluoreszenzmarker eine Bindung eingehen kann, mit mindestens einem an das Atom bzw. die Atome gebundenen Fluoreszenzmarker, der aus einem Seltenerdmetallkryptat besteht, umsetzt.

**19.** Verwendung eines Konjugats nach einem der Ansprüche 1 bis 17 als Marker.

**20.** Verwendung eines Konjugats nach einem der Ansprüche 1 bis 3 und 6 bis 16 als Nucleotidbaustein bei einer Nucleinsäure-Synthesereaktion.

**21.** Verwendung eines Konjugats nach einem der Ansprüche 1 bis 4 und 7 bis 16 zum Nachweis und/oder zur Lokalisierung von Verbindungen mit mindestens einer Nucleinsäuresequenz.

**22.** Verwendung nach Anspruch 20 bei einem Verfahren zur Bestimmung der enzymatischen Aktivität eines an einer Nucleinsäure-Synthesereaktion beteiligten Enzyms, **dadurch gekennzeichnet, daß** man die von dem Konjugat direkt oder indirekt emittierte Fluoreszenz mißt, wobei die Fluoreszenzemission mit dem Einbaugrad des Konjugats in das synthetisierte Nucleinsäure-Polynucleotid korreliert wird.

**23.** Verwendung nach Anspruch 22, **dadurch gekennzeichnet, daß** es sich bei der enzymatischen Aktivität um die Aktivität von DNA- oder RNA-Polymerase, inverser Transkriptase, Transferase oder Ligase handelt.

**24.** Verwendung nach Anspruch 20 bei einem Verfahren zur Bestimmung der enzymatischen Aktivität mit einer Nucleinsäure als Substrat.

**25.** Polynucleotid, enthaltend mehr als 1 Konjugat nach einem der Ansprüche 9 bis 16.

## Claims

**1.** Fluorescent conjugate of a nucleoside or nucleotide, **characterized in that** it comprises:

- a ribo- or deoxyribo-nucleoside or -nucleotide which is native, chemically modified or conjugated with one or more labeling molecules, in which at least one carbon atom of the ring or exocyclic nitrogen atom of the purine or pyrimidine ring or else carbon atom of the pentofuranose unit may be involved in bonding with a fluorescent marker, and
- at least one fluorescent marker bonded to said atom(s) and consisting of a rare earth cryptate.

**2.** Fluorescent conjugate of a nucleoside or nucleotide, **characterized in that** it comprises:

- a ribo- or deoxyribo-nucleoside or -nucleotide which is native, chemically modified or conjugated with one or more labeling molecules, in which at least one carbon atom of the ring or exocyclic nitrogen atom of the purine or pyrimidine ring may be involved in bonding with a fluorescent marker, and
- at least one fluorescent marker bonded to said atom(s) and consisting of a rare earth cryptate.

**3.** Conjugate according to claim 1 or 2, **characterized in that** it is a fluorescent conjugate of a nucleotide comprising a ribonucleotide selected from AMP, ADP, ATP, GMP, GDP, GTP, CMP, CDP, CTP, UMP, UDP, UTP, TMP, TDP, TTP, 2Me-AMP, 2Me-ADP, 2Me-ATP, 1Me-GMP, 1Me-GDP, 1Me-GTP, 5Me-CMP, 5Me-CDP, 5Me-CTP, 5MeO-CMP, 5MeO-CDP, 5MeO-CTP, 7-deaza-ATP and 7-deaza-GTP, or a deoxyribonucleotide selected from the deoxy- or dideoxyribonucleotides corresponding to these ribonucleotides.

**4.** Conjugate according to one of claims 1 to 3, **characterized in that** the ribo-or deoxyribonucleotide is selected from:

- 2'-deoxyuridine 5'-triphosphate or uridine 5'-triphosphate derivatives functionalized in the 5-position of the base,
- 2'-deoxycytidine 5'-triphosphate or cytidine 5'-triphosphate derivatives functionalized in the 4- or 5-position of the base,
- 2'-deoxyadenosine 5'-triphosphate or adenosine 5'-triphosphate derivatives functionalized in the 6- or 8-position of the base,
- 2'-deoxyguanosine 5'-triphosphate or guanosine 5'-triphosphate derivatives functionalized in the 6- or 8-position of the base,
- 2'-deoxy-7-deazaadenosine 5'-triphosphate or 7-deazaadenosine 5'-triphosphate derivatives functionalized in the 7-position of the base, and
- 2'-deoxy-7-deazaguanosine 5'-triphosphate or 7-deazaguanosine 5'-triphosphate derivatives functionalized in the 7-position of the base.

**5.** Conjugate according to claim 1, **characterized in that** it is a fluorescent conjugate of a nucleoside in which the ribo- or deoxyribonucleoside is selected from A, G, C, U, T, the corresponding deoxy- or dideoxynucleosides and their chemically modified analogs.

**6.** Conjugate according to claim 5, **characterized in that** the deoxyribonucleoside is selected from 3'-azido-3'-deoxythymidine and its derivatives, and the 2',3'-dideoxy analogs of A, T, C, G, U and I.

**7.** Conjugate according to any one of claims 1 to 6, **characterized in that** the fluorescent marker is bonded to a functional group introduced onto or generated on the base or on the pentofuranose unit of the ribo- or deoxyribonucleoside or -nucleotide, either directly or via a spacer arm.

**8.** Conjugate according to any one of claims 1 to 7, **characterized in that** the fluorescent marker is a terbium, europium, samarium or dysprosium cryptate.

**9.** Conjugate according to any one of claims 1 to 8, **characterized in that** the fluorescent marker is a rare earth cryptate consisting of at least one rare earth salt complexed by a macropolycyclic compound of the formula

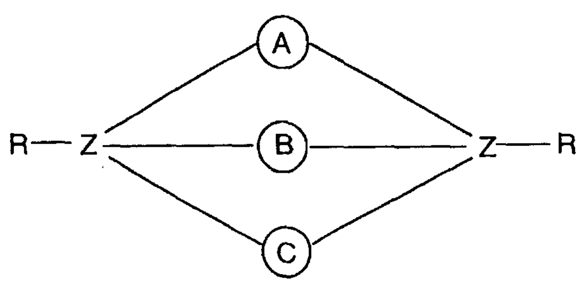

I

in which Z is a tri- or tetravalent atom, R is nothing, hydrogen, the hydroxyl group, an amino group or a hydrocarbon radical, and the divalent radicals Ⓐ , Ⓑ and © independently of one another are hydrocarbon chains which optionally contain one or more heteroatoms and are optionally interrupted by a heteromacrocycle, at least one of the radicals Ⓐ , Ⓑ and © also containing at least one molecular moiety or essentially consisting of a molecular moiety, said molecular moiety possessing a triplet energy greater than that of the emission level of the complexed rare earth ion.

**10.** Conjugate according to claim 9, **characterized in that** the fluorescent marker is a rare earth cryptate of formula (I) in which the molecular moiety is selected from phenanthroline, anthracene, benzene, naphthalene, bi- and terphenyl, azobenzene, azopyridine, pyridine, bipyridines, bis-quinolines and the compounds of the following formulae:

$$-C_2H_4-X_1-C_6H_4-X_2-C_2H_4-$$

$$-C_2H_4-X_1-CH_2-C_6H_4-CH_2-X_2-C_2H_4-$$

in which $X_1$ and $X_2$, which can be identical or different, are oxygen, nitrogen or sulfur, and

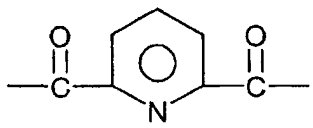

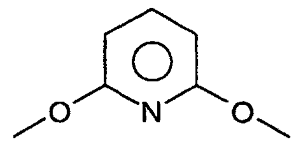

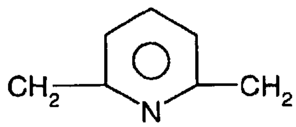

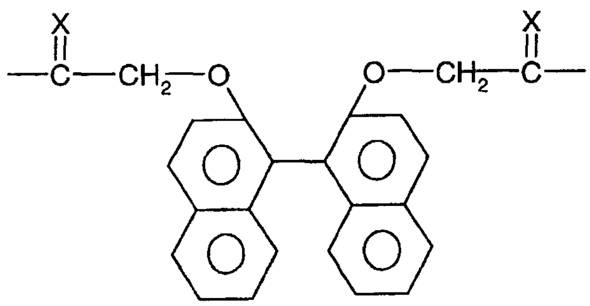

in which X is oxygen or hydrogen.

**11.** Conjugate according to claim 9 or 10, **characterized in that** the fluorescent marker is a rare earth cryptate consisting of the terbium or europium ion complexed by one of the following macrocyclic compounds:

(22)phenanthroline; (22)phenanthroline amide; (22)anthracene; (22)anthracene amide; (22)biisoquinoline; (22)biphenyl-bis-pyridine; (22)bipyridine;
(22)bipyridine amide; and tris-bipyridine, tris-phenanthroline, phenanthroline-bis-bipyridine, biisoquinoline-bis-bipyridine and bis-bipyridine diphenylbipyridine macropolycycles.

**12.** Conjugate according to claim 11, **characterized in that** the fluorescent marker is the europium cryptate Eu tris-bipyridine.

**13.** Conjugate according to one of claims 1 to 8, **characterized in that** the fluorescent marker is a rare earth cryptate consisting of at least one rare earth salt complexed by a macropolycyclic compound comprising a molecular moiety selected from bipyrazines, bipyrimidines and nitrogen heterocycles containing N-oxide groups.

**14.** Conjugate according to any one of claims 1 to 8, **characterized in that** the fluorescent marker is a rare earth cryptate consisting of at least one rare earth salt complexed by a macropolycyclic compound of formula II or III below:

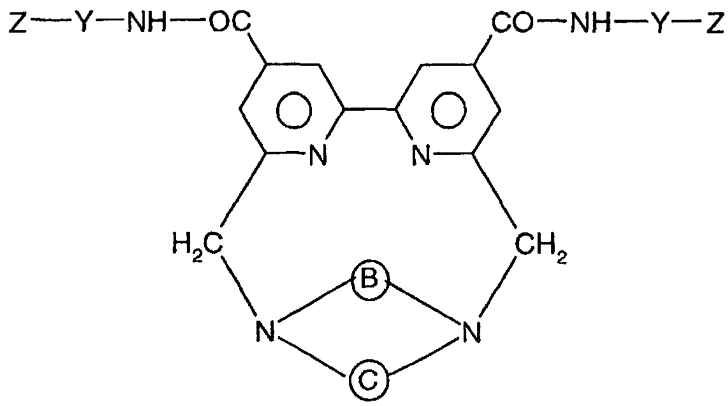

II

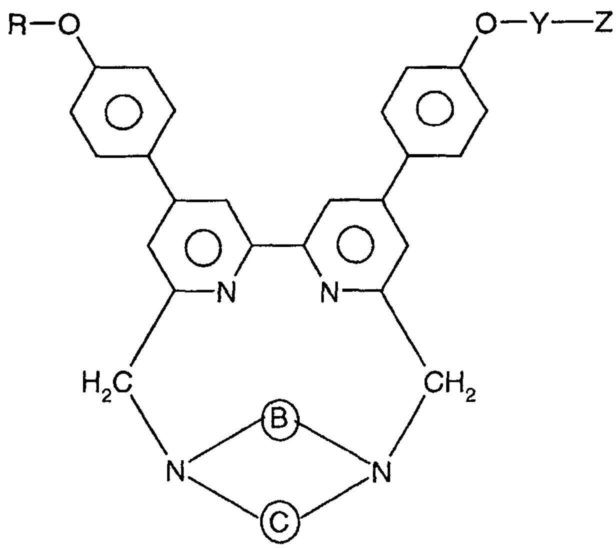

III

in which:

- the ring of the formula

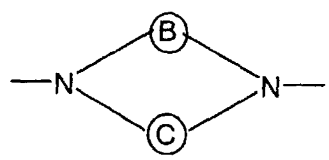

is one of the following rings:

1) 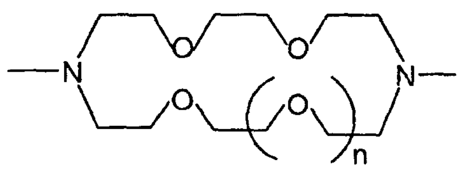

n = 0 or 1

$[N_2O_4]$ macrocycle or (22) ring

$[N_2O_3]$ macrocycle or (21) ring

2) 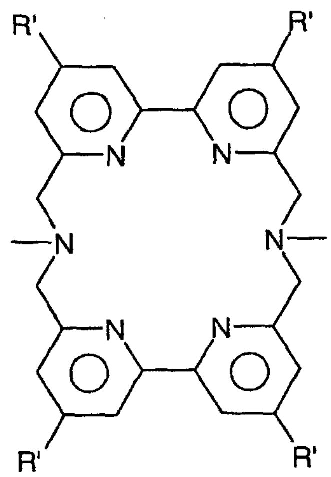

macrocycle bis-bipyridine

- Y is a group or spacer arm consisting of a divalent organic radical selected from linear or branched $C_1$ to $C_{20}$ alkylene groups optionally containing one or more double bonds and/or optionally containing one or more heteroatoms such as oxygen, nitrogen, sulfur or phosphorus, or one or more carbamoyl or carboxamido groups, from $C_5$ to $C_8$ cycloalkylene groups or from $C_6$ to $C_{14}$ arylene groups, said alkylene, cycloalkylene or arylene groups optionally being substituted by alkyl, aryl or sulfonate groups;
- Z is a functional group capable of bonding covalently with a biological substance;
- R is a methyl group or the group -Y-Z; and
- R' is hydrogen or a group -COOR", in which R" is a $C_1$ to $C_{10}$ alkyl group, preferably the methyl, ethyl or tert-butyl group, or R' is a group -CO-NH-Y-Z.

**15.** Conjugate according to any one of claims I to 14, **characterized in that** the fluorescent marker is bonded to the ribo- or deoxyribo-nucleoside or -nucleotide via a spacer arm consisting of a divalent organic radical selected from linear or branched $C_1$-$C_{20}$ alkylene groups optionally containing one or more double bonds or triple bonds and/or optionally containing one or more heteroatoms such as oxygen, nitrogen, sulfur or phosphorus, or one or more carbamoyl or carboxamido groups; $C_5$-$C_8$ cycloalkylene groups; and $C_6$-$C_{14}$ arylene groups, said alkylene, cycloalkylene or arylene groups optionally being substituted by alkyl, aryl or sulfonate groups.

**16.** Conjugate according to claim 15, **characterized in that** the spacer arm is selected from the following groups:

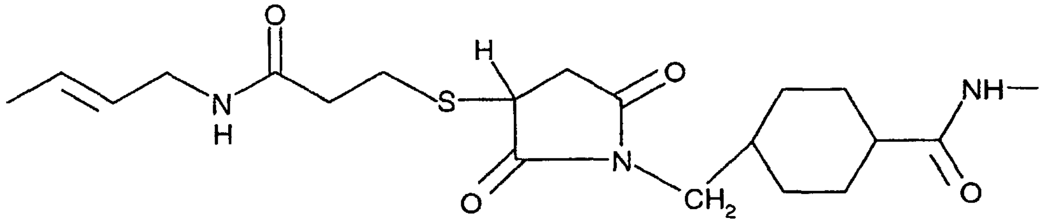

and

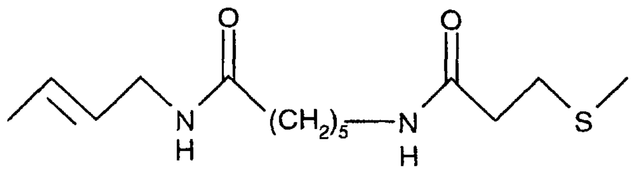

**17.** Conjugate according to one of claims 1 to 15, **characterized in that** the deoxyribonucleotide is deoxyuridine, the fluorescent marker is the europium cryptate Eu tris-bipyridine and the spacer arm is a 3-aminoallyl group.

**18.** Process for the preparation of the conjugates according to any one of claims 1 to 17, **characterized in that** a ribo- or deoxyribo-nucleoside or -nucleotide which is native, chemically modified or conjugated with one or more labeling molecules, in which at least one carbon atom of the ring or exocyclic nitrogen atom of the purine or pyrimidine ring or else carbon atom of the pentofuranose unit may be involved in bonding with a fluorescent marker, is reacted with at least one fluorescent marker bonded to said atom(s) and consisting of a rare earth cryptate.

**19.** Use of a conjugate according to any one of claims 1 to 17 as a marker.

**20.** Use of a conjugate according to any one of claims 1 to 3 and 6 to 16 as a constituent nucleotide in a nucleic acid synthesis reaction.

**21.** Use of a conjugate according to any one of claims 1 to 4 and 7 to 16 for detecting and/or locating compounds containing at least one nucleic acid sequence.

**22.** Use according to claim 20 in a method of measuring the enzymatic activity of an enzyme involved in a nucleic acid synthesis reaction, **characterized in that** the fluorescence emitted directly or indirectly by said conjugate is measured, said fluorescence emission being correlated with the degree of incorporation of said conjugate into the synthesized nucleic acid polynucleotide.

**23.** Use according to claim 22, **characterized in that** the enzymatic activity is a DNA or RNA polymerase, reverse transcriptase, transferase or ligase activity.

**24.** Use according to claim 20 in a method of measuring enzymatic activity where there is a nucleic acid substrate.

**25.** Polynucleotide comprising a number greater than one of conjugates according to one of claims 1 to 3 and 6 to 16.

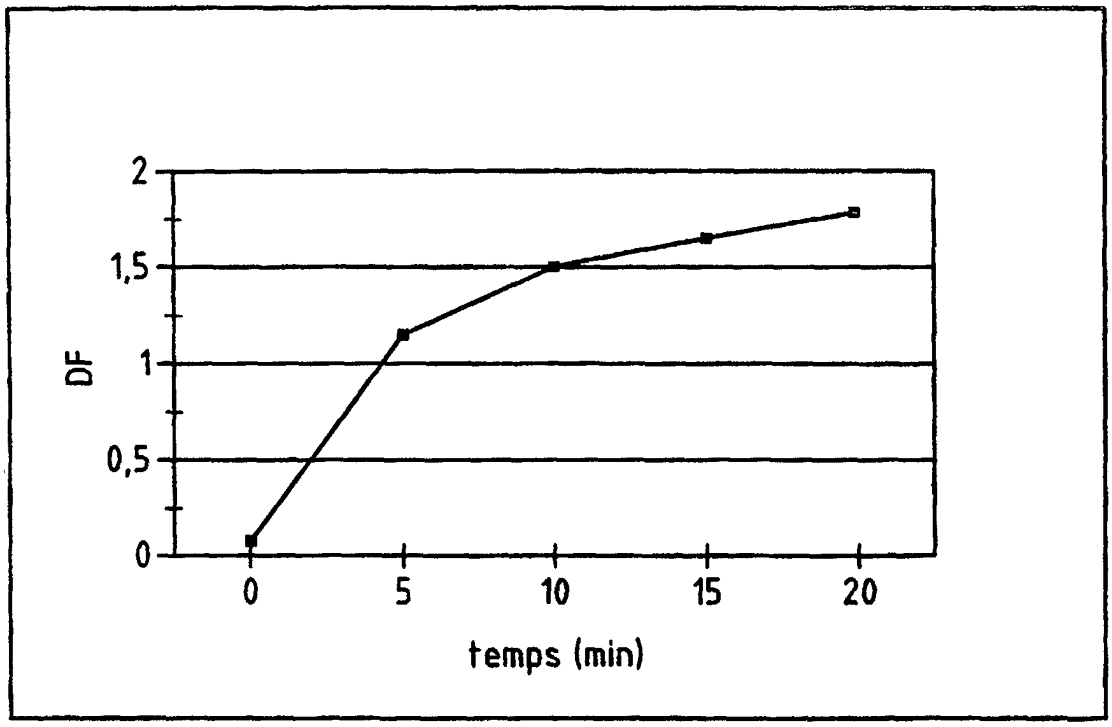


FIG.1

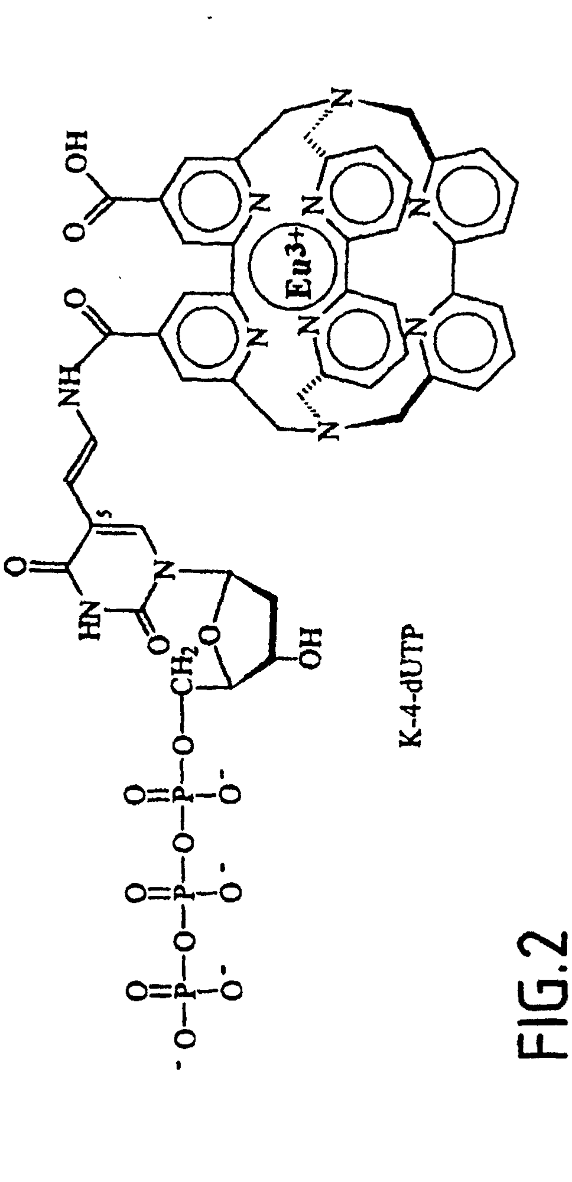

K-4-dUTP

FIG.2

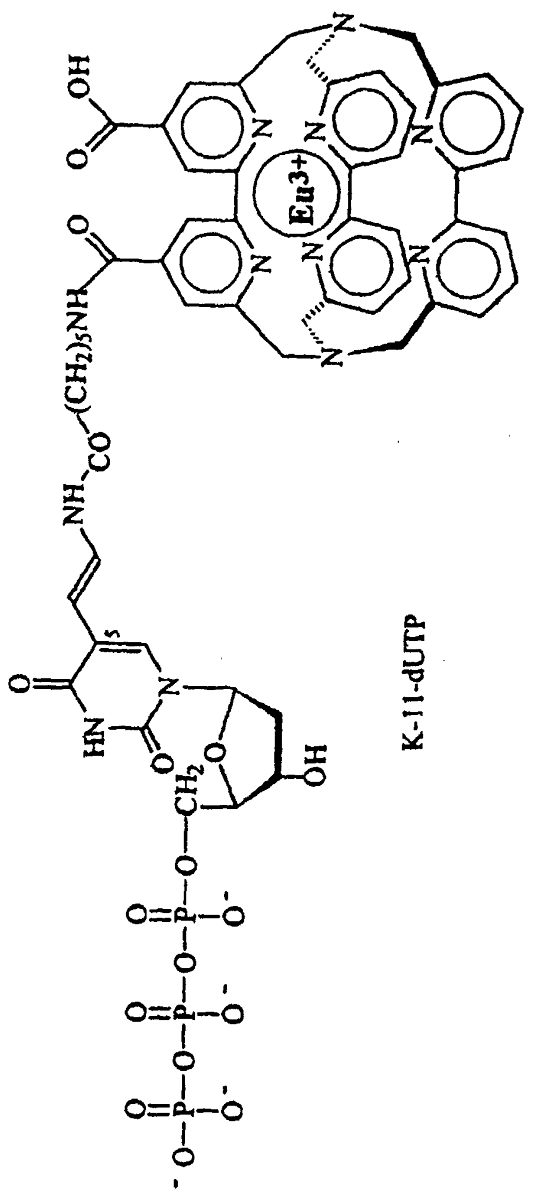

K-11-dUTP

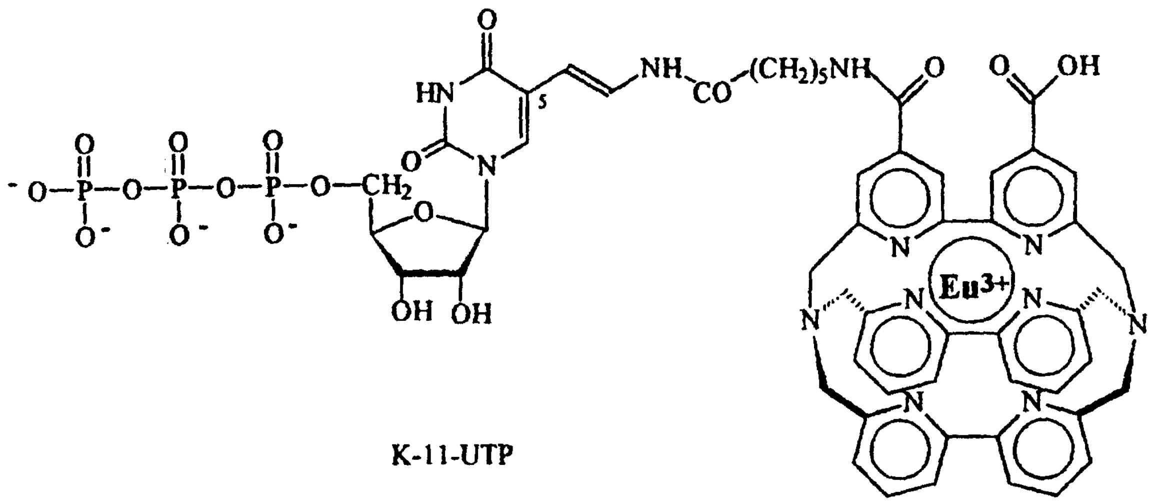

K-11-UTP

FIG.3

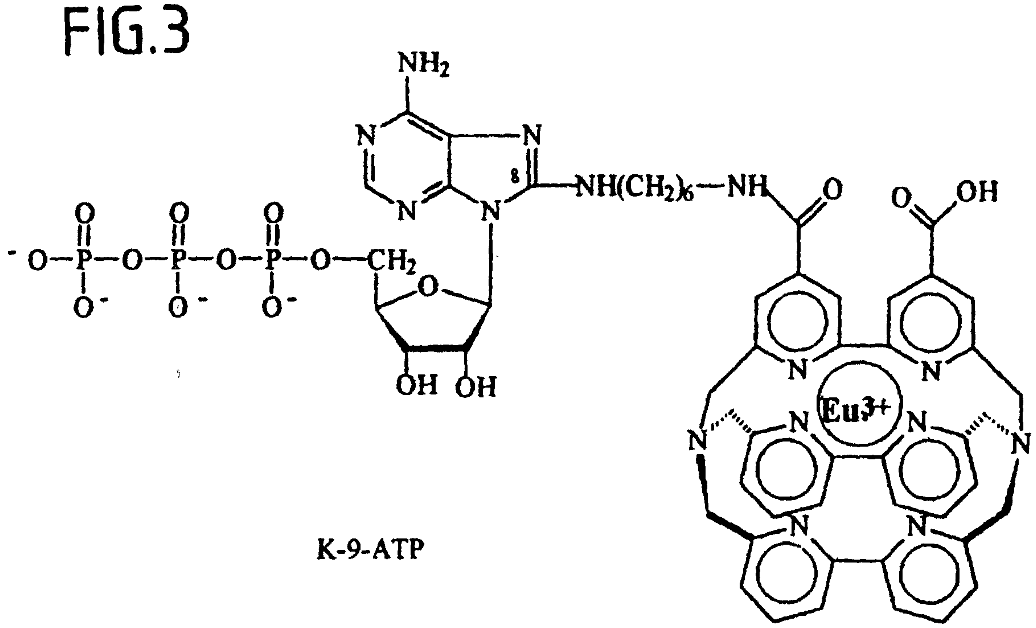

K-9-ATP

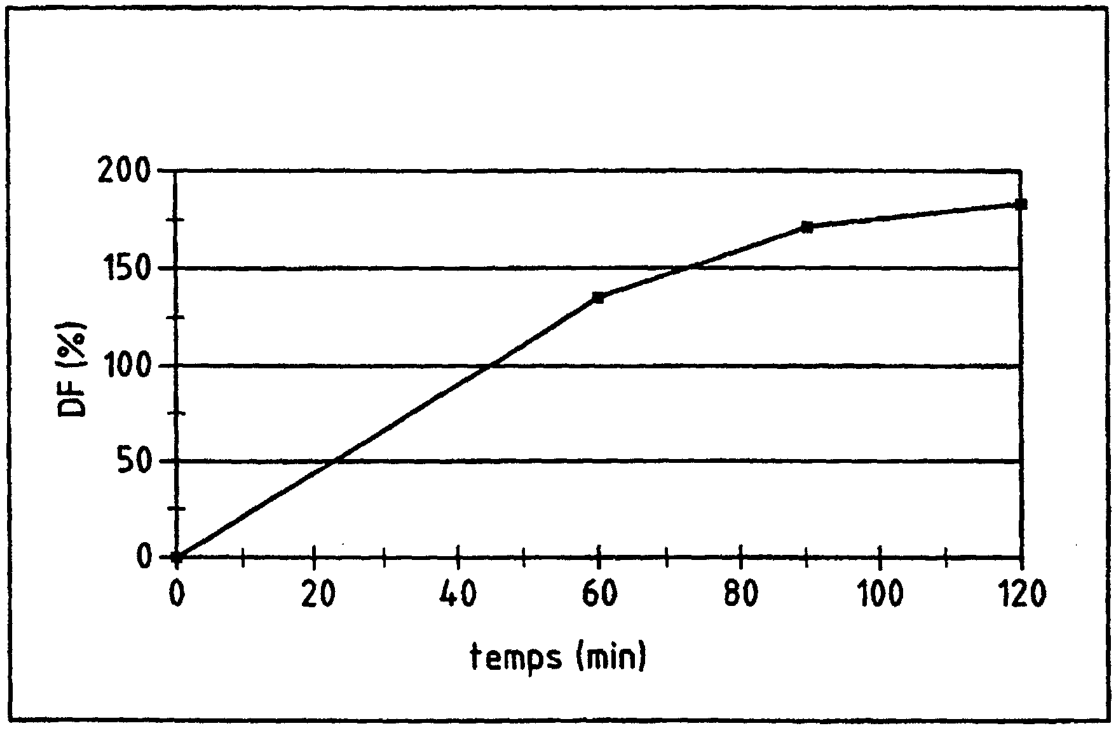


FIG.4